# EUROPEAN PATENT APPLICATION

(11) **EP 4 603 119 A1**
(43) Date of publication of application: **20.08.2025**
(21) Application number: 24195482.5
(22) Date of filing: 20.08.2024
(51) Int. Cl.: A61M 5/20, A61M 5/315, A61M 5/32, A61M 5/31

(54) **AUTOINJECTOR**

(71) Applicant: medmix Switzerland AG, 9469 Haag (Rheintal) (CH)
(72) Inventor: Morris, Anthony Paul, Balsall Common (GB); Marsh, William Geoffrey Arthur, Bonnyton, Wolverton, Stratford-upon-Avon (GB)
(74) Representative: Manitz Finsterwald Patent- und Rechtsanwaltspartnerschaft mbB

(57) **Abstract**

The present invention relates to an autoinjector (12) comprising a body (34) having a proximal end (300) and a distal end (310), with the body (34) being configured to receive a pre-filled syringe (170) having a needle (172) arranged at a proximal end (300) and a flange (174) arranged at a distal end (310); wherein the body (34) is configured to receive the pre-filled syringe (170);
wherein the body (34) further comprises a force reduction member (120) arranged between the body (34) and the pre-filled syringe (170).

## Description

The present invention relates to an autoinjector comprising a body having a proximal end and a distal end, with the body being configured to receive a pre-filled syringe having a needle arranged at a proximal end and a flange arranged at a distal end.

Autoinjectors are typically disposable devices configured to dispense medicament from a pre-filled syringe with which the autoinjector is connected. Such devices are single-use and intended for administration by a patient (i.e. self-administration) or carer.

In most of the cases, the pre-filled syringe is provided separately from the remaining parts of the autoinjector. Hence, the pre-filled syringe has to be connected with said remaining parts, mostly by inserting into a cavity provided in the body of the autoinjector. Preference is given to supplying the user with autoinjectors with pre-connected pre-filled syringes.

At point of use, the user removes, if provided, a protective cap and/or a rigid needle shield (RNS) from the proximal end of the autoinjector and positions the autoinjector at the injection site (typically the skin of the thigh or belly) and presses the autoinjector axially in a proximal direction, to achieve needle insertion of a needle of the pre-filled syringe into the skin and to initiate dispense.

Upon activation, a stopper of the pre-filled syringe is engaged by a respective part of the autoinjector. To enable a flexible system that can incorporate different pre-filled syringe fill volumes, it may be beneficial for there to be a large gap between said respective engaging part of the autoinjector and said stopper so that the engaging part of the autoinjector is able to accelerate unimpeded, potentially reaching a very high velocity before impacting said stopper. Thereby large forces can be applied to the pre-filled syringe, resulting in the worst case to damaging the pre-filled syringe.

It is an object of the present invention to make available an autoinjector in which the risk of damaging the pre-filled syringe upon activation is reduced.

This object is satisfied by an autoinjector comprising the subject matter of claim 1.

Such an autoinjector comprises a body having a proximal end and a distal end, with the body being configured to receive a pre-filled syringe having a needle arranged at a proximal end and a flange arranged at a distal end; wherein the body is configured to receive the pre-filled syringe; wherein the body further comprises a force reduction member arranged between the body and the pre-filled syringe.

In particular, said force reduction member is capable of reducing a force acting on said force reduction members by internal deformation. Said deformation can be reversible, irreversible, or even lead to destruction of the respective force reduction member. As said force reduction member is arranged within the autoinjector according to the invention between the body and the pre-filled syringe, a force applied by a part of the body onto the pre-filled syringe is transferred by and/or through the force reduction member, and thereby reduced.

The force reduction member may also be configured to aid in decelerating a syringe of the autoinjector by providing a spring feature to increase a distance over which deceleration occurs.

In summary, by the force reduction member an element providing compliance can be provided within the autoinjector, in particular in the connection of the body to the pre-filled syringe. Hence, during activation of the autoinjector, when high forces may act on the pre-filled syringe, and which are located at the connection between the pre-filled syringe and the body, these forces can be at least partly absorbed by the force reduction member. Hence, the actual force acting on the pre-filled syringe itself can be drastically lowered, and thereby the risk of damaging the pre-filled syringe upon activation can be reduced.

In this connection if should be noted that said reduction especially holds true for the peak forces and peak stresses acting on the pre-filled syringe during activation.

In this connection it should be noted that proximal end of the body, and hence of the autoinjector, is that end of the autoinjector to be placed at skin of patient, i.e., with a pre-filled syringe installed as part of the autoinjector, the end having the needle, whereas the distal end is the end remote from the skin of a patient.

Preferably, the autoinjector according to the invention can further comprise a needle guard moveable relative to the body in different states of use. More preferably, the needle guard can be mounted axially moveable with respect to other parts of the body of the autoinjector for providing a movement between a storage state, a dispensing state and a lock-out state in which states the needle guard adopts different axial positions relative to the respective part of the body. In particular, the needle guard may be configured to be axially moved in a distal direction between the storage state and the dispensing state and configured to be axially moved in a proximal direction between the dispensing state and the lock-out state.

In addition, the body of the autoinjector according to the invention may be provided as several elements connectable and connected to each other. For instance, for the actual arrangement of the pre-filled syringe, an inner body forming a component of the housing and/or a syringe carrier can be arranged and fixed to an outer body providing handling areas for the user. Preferably, said inner body can be arranged in said outer body and/or snap fit into place via a projection at the inner body cooperating with a window arranged at the outer body.

Further, the autoinjector may comprise a spring chassis arranged in the autoinjector for acting on the pre-filled syringe, especially on a stopper of the pre-filled syringe. Said spring chassis preferably may be biased with respect to the body of the autoinjector, in particular with respect to a part of the body forming a housing of the autoinjector. Further, said spring chassis may be fixed with respect to said housing part of the body for a movement relative to said housing in a storage state of the autoinjector, wherein the spring chassis being configured to axially move and act on said pre-filled syringe during dispensing.

Further additionally, or alternatively, a proximal end of the autoinjector may be covered by a protective cap. In particular, the above-mentioned needle guard may be configured to cooperate with said cap via one or more snap-fit connections, optionally with each snap fit connection comprising a protruding edge cooperating with a corresponding snap-fit area and wherein an inner surface of the housing part of the body of the autoinjector comprises one or more grooves in which one or more of said protruding edges can axially move relative to the housing on an axial movement of the needle guard. Such a cap protects an accidental activation of the autoinjector in the storage state and also prevents an accidental contact of the user with a needle thereof.

The force reduction member may be arranged at a point of contact between the body and the pre-filled syringe. At said point of contact, the pre-filled syringe is physically arranged at the body. In other words, at said point of contact, the pre-filled syringe touches the body upon arrangement. Hence, also any force applied by the body to the pre-filled syringe is acting at said point of contact. By arranging the force reduction member at said point of contact, the reduction of a force acting on the pre-filled syringe and created by an impact of the mechanism can therefore be provided with especially high efficiency. Thea risk of damaging the pre-filled syringe during usage of the autoinjector can be reduced further.

The body may comprise an abutment. An abutment is a specific formed part of the body for a physical, form-fitting arrangement of the pre-filled syringe. Preferably, the above-mentioned point of contact is provided by or arranged at said abutment. Further preferably, the abutment may be accordingly shaped with respect to the pre-filled syringe, for instance with respect to a rim and/or flange at a distal end of the pre-filled syringe.

For instance, said abutment may comprise two wings projecting distally from the body, preferably from a surface of a cavity provided in the body for the arrangement of the pre-filled syringe, for holding a respectively provided part of the pre-filled syringe, for instance a flange of the pre-filled syringe at a distal end of the pre-filled syringe.

The abutment of the body may be configured to receive the pre-filled syringe. In other words, the abutment also may provide at least partly the above-mentioned cavity of the body of the autoinjector for inserting the pre-filled syringe.

Additionally, or alternatively, the abutment of the body may be configured to receive the flange of the pre-filled syringe. Said flange of the pre-filled syringe is in most of the cases designed for providing a specific area and/or section of the pre-filled syringe for an arrangement of the pre-filled syringe on and/or in the body of the autoinjector, preferably also including a form-fitting and/or force-fitting fixation. By receiving said flange by the abutment, said arrangement, preferably fixation, can be provided more easily.

Especially for the above-mentioned feature of receiving of the flange of the pre-filled syringe, the abutment may comprise the above-mentioned two wings.

The body may comprise an inner body and an outer body. By providing the body divided in separate parts, in particular at least as an inner body and an outer body, a production of the body in a shape otherwise not possible can be provided. The two separate parts are produced, in particular by injection molding, and afterwards arranged on each other to form the body. The inner body may be arranged in the outer body and snap fit into place via a projection at the inner body cooperating with a window arranged at the outer body. Both, the inner and outer body, respectively, can be adapted to specific functions. As an example, the inner body can be configured for the arrangement of the pre-filled syringe, for instance by providing a cavity for said arrangement or the above-mentioned abutment. The outer body on the other hand, can be for instance provide handling areas for the user of the autoinjector.

The force reduction member may be arranged at the distal end of the inner body. In this embodiment, the pre-filled syringe is arranged at the inner body of the autoinjector. As the force reduction member acts between the body, namely the inner body, and the pre-filled syringe, and after the distal end of the inner body is furthest away from the patient, an arrangement space or volume for the pre-filled syringe can be maximized by providing the force reduction member at said distal end of the inner body.

The force reduction member may comprise one or more bridges. As a bridge in the sense of the present invention may be considered a connecting structure which comprises a reduced cross section compared to sections of elements connected by said bridge. Said bridge as a structure with reduced cross section is especially capable of reducing a force acting on said force reduction members by internal deformation.

In general, during usage of the autoinjector, the one or more bridges preferably fulfil two different tasks. First, the one or more bridges preferably are designed to help maintain rigidity and stability during manufacture of the autoinjector, in other words when applied loads are relatively low. The manufacture of the autoinjector encompasses, but is not limited to, support measurement inspections and/or assembly. Second, the one or more bridges preferably are designed to break when a load acting on the one of the one or more bridges exceeds a certain limit. in other words, the bridge of the force reduction member additionally acts as a pre-determined breaking point up to which kinetic energy is absorbed by the bridge and hence helps reducing peak forces and peak stresses generated during usage of the autoinjector.

The force reduction member may further comprise a peak force reduction structure and a support. In other words, the force reduction member comprises dedicated parts for arranging and/or fixing the force reduction member within the autoinjector, namely the support, and for the actual reduction of acting forces, namely the peak force reduction structure. By that, each of said structures can be specifically constructed for its respective function. Compromises in the design of these dedicated parts, which could lead to restrictions in functionality, can thus be avoided.

A respective one of the one or more bridges may be arranged between the peak force reduction structure and the support. By that, the actual force reduction provided by the force reduction member can be improved, as not only the peak force reduction structure, which is specifically constructed for said force reduction, but also the one or more bridges take part in the force reduction. In particular, the one or more bridges may be arranged as pre-determined breaking points between the peak force reduction structure and the support.

The force reduction member may be a spring element. Spring elements can be provided in different shapes and stiffnesses, such as for instance but not limited to a leaf spring or a spiral spring, providing a vast variety of providable force reduction. Especially, said spring elements are very easy elements for providing compliance within any structure. Further, spring elements are flexible and reversibly deformable, at least as long as not overstretched.

The force reduction member may be arranged between the abutment and a main body of the inner body. As mentioned above, the abutment is a specific formed part of the body for a physical, form-fitting arrangement of the pre-filled syringe. Preferably, both the abutment and the force reduction member can be part of the inner body, wherein the force reduction member is arranged between the abutment and the main body of the inner body, both elements forming the distal end of the inner body. By providing said force reduction member between the abutment, which the pre-filled syringe form-fittingly contacts, and the main body of the inner body, a force acting on the syringe, in particular induced by an activation of the autoinjector, can be reduced. In particular, it can be prohibited that the user acts a force on the pre-filled syringe which exceeds a structural stability of the pre-filled syringe

The force reduction member may comprise first and second struts connected by a web. Preferably, the struts are arranged parallel or at least essentially parallel to each other, and the web is arranged in between. In other words, a slight movement of said structure perpendicular to the extension of the struts is possible, wherein the web ensures an essential constant distance between the struts. Said movement may cause a deformation at fixing points of at least one of the struts, thereby deducing deceleration and hence reducing a force acting within the autoinjector.

The first strut may be connected to the abutment via two first points of connection. By that, any force acting on the abutment can be transferred into and/or by the first strut, and hence further on into the complete structure formed by the first and second strut connected by the web. By providing two points of connection, unwanted torque acting on the first strut can be reduced, preferably prevented.

The web may be arranged on a side of the first strut remote from the two first points of connection. By this, connecting the second strut to the web is not hindered by the points of connection to the abutment. The above-mentioned structural benefits provided by the first and second strut connected by the web can thereby be provided more easily.

The second strut may be connected to the body via two second points of connection. As already described above with respect to the two points of connection of the first strut to the abutment, similarly any force acting on the body of the autoinjector, if applicable preferably the inner body of the autoinjector, can be transferred into and/or by the second strut, and hence further on into the complete structure formed by the first and second strut connected by the web. By providing two points of connection, unwanted torque acting on the second strut can be reduced, preferably prevented.

In particular, the structure comprising a first and second strut connected by a web can be arranged between the body and the abutment. As mentioned above, the structure comprising the two struts connected by the web can absorb and thereby reduce an acting force. Further, the pre-filled syringe may be form-fittingly arranged at the abutment. Thereby, a force acting between the body and the pre-filled syringe can be reduced, preferably prevented, more easily.

At least one of the one or more bridges may be arranged between the first strut and the support. As a structure with reduced cross section, said one of the one or more bridges is especially capable of reducing a force acting between the first strut and the support. In particular, said one of the one or more bridges can act as a pre-determined breaking point upon acting of a force. A force too high to be absorbed by the structure formed by the first and second strut connected by the web can thereby be compensated by the breaking of the one or more bridges. Preferably, two bridges are provided that are each arranged between a respective end of the first strut and the support.

Further, at least one of the first and second struts may be U-shaped in geometry. Preferably, both struts may be U-shape, more preferably adapted to each other. By this shape, the struts encompassing a space in which the pre-filled syringe can be arranged. An overall more compact design of the autoinjector can thereby be provided.

The autoinjector may further comprise the pre-filled syringe arranged within the body. All features described above with respect to the autoinjector according to the present invention can be provided especially with the pre-filled syringe arranged in the body of the autoinjector.

According to a further aspect of the present invention the drug delivery device may comprise an inner body and a spring chassis moveable relative thereto, wherein the spring chassis comprises a first end of dose click member and the inner body comprises a complementary shaped second end of dose click member interacting with the first end of dose click member, wherein one of the first and second end of dose click members comprises an end of dose click ramp and the other one of the first and second end of dose click members comprises an end of dose click arm, wherein one or more further ramp features are provided in addition to the end of dose click ramp that can also interact with the end of dose click arm. Such further features aid in guiding the end of dose click arm such that the end of dose click reliably occurs. However, their main purpose is to reduce force losses by avoiding unintentional contact of the end of dose arm with other elements of the autoinjector such as for instance a drive spring.

According to a further aspect of the present invention the drug delivery device may comprise an inner body and a spring chassis moveable relative thereto, wherein the spring chassis comprises a first end of dose click member and the inner body comprises a complementary shaped second end of dose click member interacting with the first end of dose click member, wherein one of the first and second end of dose click members comprises an end of dose click ramp and the other one of the first and second end of dose click members comprises an end of dose click arm, wherein the end of dose click members are configured to make an end of dose click sound before the end of dose click arm contacts one or more further ramp features. Such further features aid in guiding the end of dose click arm such that the end of dose click reliably occurs.

It is to be noted that the drug delivery device may be an autoinjector, in particular an autoinjector according to the above presented aspect of the present invention.

Two further ramp features may be provided one on each side of the end of dose click ramp. In this way the end of dose click arm can be more reliably guided.

The end of dose click ramp may have a curved extent. Such shapes are comparatively simple to manufacture in an injection molding process.

The end of dose click ramp and the one or more further ramp features may be arranged at a common base. Both elements, namely the end of dose click ramp and the one or more further ramp features, respectively, are provided for physically interacting with the end of dose click arm, and hence are preferably arranged adjacent to each other. By providing a common base for arranging these elements, the overall design of the drug delivery device can be simplified, especially concerning manufacture in an injection molding process.

The end of dose click ramp may extend further in the axial direction than each of the one or more further ramp features. Thereby, an engagement by the end of dose click arm of the end of dose click ramp prior to a respective engagement of the one or more further ramp features can be ensured. As said engagement of the end of dose click ramp is essential for the actual end of dose click, the occurrence of the end of dose click can be ensured further.

The end of dose click ramp and the one or more further ramp features may be provided on an inner surface of the spring chassis. The spring chassis and the inner body are moveable with respect to each other upon activation of the drug delivery device. As already mentioned above, both elements, namely the end of dose click ramp and the one or more further ramp features, respectively, are provided for physically interacting with the end of dose click arm, and hence are preferably arranged adjacent to each other. By providing these elements on an inner surface of the spring chassis, said adjacent arrangement can be provided easily.

The end of dose click arm may taper towards an end thereof. Upon activation, the end of dose click arm and the end of dose click ramp are moved towards each other. Providing the end of dose click arm with a tapered end makes it easier for these components pass against each without catching or jamming. The same holds true for respective one of the one or more further ramp features contacted by the preferably tapered end of the end of dose click arm.

The end of dose click arm may be provided within the inner body. Preferably, the end of dose click arm may be integrally formed at the inner body. The spring chassis and the inner body are moveable with respect to each other upon activation of the drug delivery device. As mentioned above, the end of dose click ramp may be arranged at the spring chassis. By providing the end of dose click arm within the inner body, preferably by integrally forming the end of dose click arm at the inner body, a relative motion of the end of dose click arm and the end of dose click ramp with respect to each other can be provided in an especially simple manner.

The end of dose click arm may comprise one or more shoulders. Preferably, said one or more shoulders are constructed to run on a respective one of the one or more further ramp elements. Engaging said one or more further ramp elements by the end of dose click arm during activation of the drug delivery device can thereby be provided in an especially simple and simultaneously safe way.

The end of dose click arm may comprise two shoulders. Preferably, said two shoulders are positioned on either side of a tapered end of the end of dose click arm. Thereby, two further ramp elements likewise positioned on different sides of the end of dose click ramp can be engaged, preferably simultaneously. In addition, any unwanted torque caused by uneven contact of the shoulders with the respective one of the one or more further ramp elements can be avoided.

The spring chassis may be configured to move towards the inner body. As described above, the spring chassis and the inner body are moveable with respect to each other upon activation of the drug delivery device. In this preferred embodiment, the inner body stays fixed within the drug delivery device, and the spring chassis moves, in particular towards the inner body. In the sense of the present invention, the movement of the spring chassis towards the inner body may also include a movement of the spring chassis within the inner body, wherein preferably said movement is along a common longitudinal extension of both elements involved. The inner body may for instance also provide part of the handling areas of the drug delivery device. Hence, upon activation of the drug delivery device the inner body stays fixed in the perception of the user, and the spring chassis moves internally within the drug delivery device.

The end of dose click members may be configured to make an end of dose click sound before the one or more shoulders of the end of dose click arm contact the one or more further ramp features of the first member. The end of dose click occurs by the interaction of the end of dose arm and the end of dose ramp, preferably by that the end of dose click arm snaps behind the end of dose click ramp at the end of its movement along the end of dose click ramp. By ensuring a contact between the one or more shoulders and the respective one of the one or more further ramp elements after said occurrence of the end of dose click, an unwanted interference with the provision of said end of dose click can be avoided. In addition, also an unintentional contact of the end of dose click arm with other elements of the autoinjector such as for instance a drive spring, can be avoided.

The drug delivery device may comprise a pre-filled syringe, with the spring chassis comprising a piston rod acting on a stopper of the pre-filled syringe and the drug delivery device being configured to emit an end of dose click sound when a medicament stored in the pre-filled syringe is being dispensed from the pre-filled syringe. All features described above with respect to the drug delivery device according to the present invention can be provided especially with the pre-filled syringe arranged in the drug delivery device, wherein said drug delivery device is constructed and configured to emit said end of dose click sound.

The drug delivery device may be configured to emit the end of dose click sound when a medicament stored in the pre-filled syringe is near completely or completely dispensed from the pre-filled syringe. In the sense of the present invention, near completely dispensed preferably means that more than 75%, more preferably more than 90%, even more preferably more than 95%, of a medicament previously stored in the pre-filled syringe is dispensed. An information to the user that the dose delivery process is nearly completed can thereby be provided.

Alternatively, or additionally, according to a further aspect of the present invention the drug delivery device may comprise a component of the housing in which a drive mechanism is stored at least partly within the component of the housing and guidable in one direction of movement for dispensing a material stored within the drug delivery device, wherein the component of the housing comprises one or more engagement members on an inner surface thereof, with the one or more engagement members respectively facing the drive mechanism and being engagable by an element of the drive mechanism.

In some embodiments of drug delivery devices, it is possible that the drive mechanism accelerates to high speeds, and hence gaining considerable kinetic energy, in particular before the actual dispensing process starts. This in turn can lead to the generation of very high stress in components of the drug delivery device, in particular if applicable in a pre-filled syringe. By slowing down the movement of the drive mechanism, said stress and in particular damage caused by said stress can be avoided.

By providing the one or more engagement members, the one or more engagement members are engaged by the drive mechanism during activation of the drug delivery device, in particular preferably before the actual dispensing starts. Hence, at least part of the mechanical energy provided by the drive mechanism is diverted and is used for the engagement of the one or more engagement members. This is of advantage, as the actual dispensing act has not started yet, and said provided mechanical energy, if not diverted, might lead to an excessive large acceleration of the drive mechanism. Preferably, the engagement members are configured to slow down an axial motion of the drive mechanism, by converting the axial motion at least partially into an oscillating transverse motion. An unpleasant high acceleration within the delivery device upon activation of the drive mechanism can thereby be avoided.

It is to be noted that the drug delivery device may be an autoinjector, in particular an autoinjector according to the above presented aspect of the present invention.

The housing of the drug delivery device may be provided by an inner body and/or an outer body of the drug delivery device.

The drive mechanism may be provided as a spring chassis.

Two engagement members may be provided, with the two engagement members being arranged on oppositely disposed inner surfaces of the component of the housing. By that, the drive mechanism can be arranged between the said two engagement members, and an engagement of the drive mechanism with both engagement members can be provided easily.

The one or more engagement members may be configured to deflect the element of the drive mechanism transversely and/or radially with respect to said one direction of movement. Preferably, the one or more engagement member may be provided with an extension along the one direction of movement. By deflecting the element of the drive mechanism transversely and/or radially with respect to said one direction of motion, slowing down an axial motion of the drive mechanism along said one direction of motion can be efficiently provided.

The engagement members may comprise an outer surface having an undulating structure comprising peaks and valleys. Such an undulating structure is especially capable of inducing the above described oscillation in the drive mechanism by periodically deflecting the element of the drive mechanism.

The peaks and valleys may be peaks and valleys of a wavelike and/or saw tooth like structure.

The peaks and valleys of one of the engagement members may be offset with respect to the peaks and valleys of the other one of the engagement members along the one direction of movement. In other words, the two engagement members are arranged adapted to each other, wherein perpendicular to the one direction of motion a peak of one engagement member corresponds to a valley of the respective other engagement member, and vice versa. By that, the oscillations induced by the two engagement members mutually reinforce each other.

The one or more engagement members may be provided to reduce a speed of the drive mechanism in the one direction of movement. High speeds may lead to undesired high mechanical stress on components of the drug delivery device upon contact between the drive mechanism and stopper. By reducing said speed of the drive mechanism, this can be prohibited.

Preferably, the one or more engagement member limits the speed of the drive mechanism in the one direction of movement, and hence prevent said speed from exceeding a certain threshold.

The drive mechanism may have one or more complementary shaped counter engagement members, such as cut outs, formed at one or more positions corresponding to a position of the one or more engagement members. Said counter engagement members are intended for engaging a respective one of the one or more engagement members provided by the housing. An effectivity of the slowing down process can thereby be provided.

The two oppositely disposed engagement members may face one another and/or face in opposite directions. In both cases, an engagement of said engagement members with the respective elements of the drive mechanism can be provided in an adapted way in such that the slowing effects of both engagement members reinforce each other.

The drug delivery device may be a single use autoinjector. The drug delivery device, especially the autoinjector, may further comprise a pre-filled syringe, with the pre-filled syringe comprising a stopper and the drive mechanism acting directly on the stopper. All advantages described above can also be provided for an autoinjector or a drug delivery device comprising a pre-filled syringe, comprising the above described features capable of slowing down a movement of the drive mechanism along the one direction of movement.

The drug delivery device may be a spring driven drug delivery device comprising a drive spring. In other words, the activation of said drug delivery device is purely mechanical, and no open or closed loop control of the movement of the drive mechanism is present. However, by providing the one or more engagement members and the respectively connected features described above, all advantages concerning limiting a speed of the driving mechanism described above can also be provided for a spring driven drug delivery device.

The drive mechanism may comprise a spring chassis. Spring chassis are devices known to be used for constructing drive mechanisms of drug delivery devices. Hence, by implementing the features described above connected to the one or more engagement members, also spring chassis can be provided with the respectively described advantages.

The one or more engagement members may be configured to bring about an oscillation of the spring chassis with respect to said one direction of movement. An oscillation in the sense of the present disclosure is not necessarily periodic, but might be any movement which comprises alternating acceleration in opposite directions, preferably perpendicular to the one direction of movement. For an actual periodic oscillation, periodic acceleration in opposite directions is needed. However, random opposite accelerations caused by uneven and/or non-periodic engagement members might lead to a more chaotic oscillation without a constant phase and/or amplitude. As already mentioned, the direction of the oscillation preferably is perpendicular or at least essentially perpendicular, to said one direction of motion, a repeated and hence essentially continuous drain in movement energy from the axial motion of the drive mechanism can be provided. In other words, slowing down said axial motion is enhanced.

The spring chassis may comprise a piston rod. Said piston rod is capable and constructed to engage a pre-filled syringe, preferably a stopper of a pre-filled syringe, for delivery of a medicament stored in the pre-filled syringe.

The spring chassis may be an at least generally U-shaped spring chassis. Thereby, the two main functions of the drive mechanism, namely providing an actuation force and acting on for instance a pre-filled syringe for causing delivery of a medicament, can be provided by different and distinct parts of the spring chassis, namely the two limbs of the U-shape. Preferably, Unwanted interference between these two functions can be avoided. In particular, the U-shape allows arranging a physical barrier between the limbs of the U-shape, for instance provided by a body and/or housing of the drug delivery device.

In particular, the piston rod may form one first limb of the U-shaped spring chassis and/or the drive spring is accommodated in a second limb of the U-shaped spring chassis. Each of these two embodiments, which are nevertheless preferably provided in combination, can ensure that the source of the driving force, namely preferably the drive spring, acts directly on the container providing the medicament to be delivered, namely a pre-filled syringe.

A respective engagement member may interact with a respective limb of the at least generally U-shaped spring chassis. In other words, each limb interacts with a respective one of the engagement members. A one-sided load acting on the U-shaped spring chassis caused by interacting with the respective engagement members can thereby be avoided.

Alternatively, or additionally, according to a further aspect of the present invention the drug delivery device may comprise a piston rod that is stored at least partly within the drug delivery device and guidable in one direction of movement for dispensing a material stored within the drug delivery device, the piston rod comprising a force transmitting end, a force receiving end and a connecting portion arranged between the force transmitting end and the force receiving end, wherein the at least a part of the connecting portion has a reduced cross-sectional area with respect to at least one of the force transmitting end and the force receiving end. During activation, high compressive forces can act on the piston rod, causing the piston rod to bend. By providing a connecting portion comprising an area with a reduced cross section, a risk of contact of at least said area of the connecting portion with an inner surface of a pre-filled syringe, in which the piston rod engages during activation, can be drastically reduced.

The connecting portion may have a radial cut-out defining one or more radial portions with reduced diameter. Such shapes are comparatively simple to manufacture in an injection molding process.

The drug delivery device may be spring driven and comprises a drive spring acting on the force receiving end. Springs are mechanically simple elements, which nevertheless are capable of reliably providing the mechanical energy and/or force necessary for actuating the delivery process.

The piston rod may have a piston rod axis and the spring has a drive spring axis and the piston rod axis and the drive spring axis are in parallel to one another and offset from one another with respect to piston rod at the force receiving end. In other words, upon activation of the drug delivery device the spring acts at least indirectly on the force receiving end. When the piston rod meets the stopper of the syringe, this spring force causes an axial load which might lead to bending via buckling. The offset of the drive spring axis and the piston rod axis might cause a torque to act on the piston rod causing an additional movement and/or deformation of the piston rod, in particular of the connecting portion. This might further encourage buckling and thereby the above mentioned bending. By the provided at least partly reduced cross-sectional area of the area of the connecting portion, said movement and/or deformation can be anticipated, and an unwanted and possibly harmful contact of the connecting portion to other parts of the drug delivery device, including in particular a pre-filled syringe, can be avoided.

The drug delivery device may further comprise a reservoir filled with a medicament and comprising a stopper arranged in the reservoir, wherein the force transmitting end acts on said stopper. Said reservoir can be for instance a pre-filled syringe. With the design of the connection portion according to this embodiment of the present invention, in particular by the provided at least partly reduced cross-sectional area of the area of the connecting portion, an unwanted and possibly harmful contact of the connecting portion to said reservoir can be avoided.

The force transmitting end may directly act on said stopper. In other words, during delivery the piston rod moves, in particular pushes, the stopper into the reservoir, and thereby the medicament is conveyed and hence delivered at the opposing end of the reservoir.

The force transmitting end that acts on said stopper may have a diameter of more than 80% of said stopper. An especially good physical contact, and hence a transmission of a delivering force, between the piston rod and the stopper can thereby be provided. Preferably, the force transmitting end may act on more than 82%, preferably more than 85% of the diameter of said stopper.

The drug delivery device may further comprise a pre-filled syringe comprising said reservoir. A pre-filled syringe comprises next to the reservoir further components needed for an application of the medicament, especially an injection needle. Hence, by using a pre-filled syringe as part of the drug delivery device, a ready-to-use status of the drug delivery device can be achieved.

It is to be noted that, if the reservoir is provided as part of a pre-filled syringe with a needle, preferably also a needle guard is provided, covering the needle before usage of the drug delivery device.

The reduced diameter of the connecting portion may be reduced by at least about 5% with respect to at least one of the force transmitting end and the force receiving end. Preferably, the reduced diameter of the connecting portion may be reduced by at least about 10%, more preferably by at least 25%, with respect to at least one of the force transmitting end and the force receiving end, in particular with respect to the smaller one of the two respective ends of the piston rod. The effect of avoiding unwanted contact with other elements of the drug delivery device upon activation due to an undesired movement and/or deformation of the piston rod can thereby be provided more easily.

The one radial cut-out may extend over 30 to 70 % of a length of the piston rod. Preferably, the one radial cut-out may extend over 40 to 60 %, especially 45 to 55% of a length of the piston rod. In particular, said extension of the radial cut-out may be adapted to the expected undesired movement and/or deformation, if applicable for instance based on a strength of the implemented spring. A suitable compromise between avoiding any unwanted contact and reducing structural stability and/or stiffness by reducing the cross-sectional area can be provided.

The radial cut-out may have chamfered ends. Sharp edges and/or corners, in particular comprising an angle of 90°, may lead to high stress in the respective material at said edges and/or corners. By providing chamfered ends for the radial cut-outs, said sharp edges and the accompanied problems can be avoided.

The connecting portion comprises one or more guide elements arranged over its length. As mentioned above, reducing a cross-sectional area may lead to a reduced structural stability and/or stiffness. By adding one or more guiding elements arranged over its length, this problem can be addressed.

Preferably, the guide elements may comprise a transverse wall and one or more noses distributed over a length of the connecting portion. As the wall is transverse, a stiffness of the piston rod, especially at the portion with reduced cross-sectional area, is automatically enhanced. Said noses may provide further guidance by contacting a part of the drug delivery device other than the reservoir, for instance a rib provided on an inner surface if a housing, for providing guidance of the piston rod before entering the reservoir.

The piston rod may be an integral part of an at least generally U-shaped spring chassis, with the piston rod forming one limb of the U. The other limb may be used for exerting the force onto the piston rod necessary for the dispensing process. A separation of the force exerting part and the actual acting on the stopper of the reservoir can thereby be achieved, avoiding for instance direct contact of the spring and the reservoir. In particular, the two limbs of the U-shaped spring chassis may even engage a separating wall in between.

The drive spring may be partly received in the other limb of the U. Received in the sense of the present invention especially encompasses that the respective limb of the U comprises a specific volume for receiving the spring at least partly, and/or that the drive spring encompasses a respective section of the limb of the U. A mechanically secure connection between the drive spring and the spring chassis comprising the piston rod as respective other limb can thereby be provided.

The drug delivery device may comprise the pre-filled syringe filled with material. All features described above with respect to the drug delivery device according to the present invention can be provided especially with the pre-filled syringe arranged in the drug delivery device, in other words with the drug delivery device comprising the pre-filled syringe.

Alternatively, or additionally, according to a further aspect of the present invention a metal insert for an autoinjector, the metal insert comprising a cylindrical body having a first end and a second end, the metal insert further comprising one or more projections projecting axially from the second end of the body and comprising one or more barbs extending radially outwardly. Said barbs allow an easy but secure fastening of the metal insert within the autoinjector.

Preferably, said metal insert can be arranged in a cap of the autoinjector, providing the feature of a remover for a RNS of a pre-filled syringe arranged in the autoinjector. However, if needed such a metal shield can be used in any embodiment of a drug delivery device.

The projections may be evenly distributed circumferentially at the second end of the body. An especially firm fixation of the metal shield within the autoinjector can thereby be provided. In particular, a torque acting on the metal insert due to an uneven fixation, which may lead to a tilting of the metal insert, can be avoided.

At least one of the one or more projections may comprise two barbs arranged at circumferentially opposing ends of the respective projection. In other words, the two barbs extend axially on opposing edges of the respective projection. This allows arranging and providing two barbs on a single projection in a maximized distance to each other, enhancing the ability provided by the barbs to fasten the metal insert within the autoinjector.

The barbs may be tapered towards the first end. Tapered towards the first end in the sense of the present invention is to be understood that a radial extension of the respective barb gets smaller towards the first edge. Especially for embodiments in which the metal insert is inserted with its first end first into an arrangement volume of the respective autoinjector, the tapered barbs do not hinder said arrangement process, but nevertheless ensure secure fixation against movement of the metal insert in the opposite direction, namely towards its second end.

The metal insert may further comprise flaps extending radially inwardly towards the first end. Hence, the flaps protrude into the inner volume of the cylindrical metal insert, wherein the front ends of said flaps are facing the first end. If the metal insert is used in a cap of an autoinjector as a remover for a RNS of a pre-filled syringe arranged in the autoinjector, said front ends of the flaps protrude towards the end of the cap. Thereby, the flaps can engage with respective features of the RNS, or simply dig into a plain wall of the RNS. Removing the RNS when removing the cap with the metal insert can thereby be provided.

The flaps may all be arranged at the same axial height. Thereby all flaps act on the RNS at the same height. An especially even force acting on the RNS for removing the RNS can thereby be provided, avoiding any unwanted torque which may lead to tilting.

Two or more groups of flaps may be provided, with the different groups of flaps being arranged at different axial heights. As mentioned above, the flaps may engage with respective features of the RNS. By providing different groups of flaps being arranged at different axial heights, the metal insert can be used for removing different types of RNS on which said respective features are provided at different axial positions.

Between 3 and 12 metal flaps may be provided. Preferably, the flaps are evenly distributed circumferentially on the inner surface of the metal insert. With 3 or more flaps, a secure and especially even grip on the RNS can be provided, without any unwanted torque which may lead to tilting. Further, by providing at maximum 12 flaps, an excessive reduction in structural integrity of the metal insert by the flaps can be avoided.

Between 3 and 4 flaps may be provided per group of flaps. As mentioned in the previous paragraph, 3 or more flaps, a secure and especially even grip on the RNS can be provided, without any unwanted torque which may led to tilting. By limiting the number of flaps per group to 4 or less, providing more groups can be provided, thereby enhancing the number of different RNS to which the respective metal insert is compatible with.

A front end of the flaps may be flat with rounded edges. In other words, the flaps are provided with rounded edges, preferably even with deburred edges. A risk of unwanted harm to any user of the metal insert, for instance during mounting into a cap, can be reduced. Simultaneously, engaging with respective features of the RNS can is still ensured.

An axial height of the metal insert may be greater than a width of the insert measured between oppositely disposed barbs. RNS are known to be elongated elements comprising an essentially cylindrical and/or truncated cone shape. By providing the metal insert with an axial height greater than its width, an adaptation of the metal insert to the expected shape of an RNS can be provided.

The metal insert may be formed from sheet metal. Forming cylindrical metal inserts from sheet metal is comparatively simple to manufacture.

The metal insert may be configured to be inserted into a cap of an autoinjector. For that the metal insert is arranged and fixed in the cap by the barbs provided on the one or more projections of the metal insert. In said cap, the metal insert may be used for removing an RNS. Flaps protruding axially inward from an inner surface of the metal insert can be used for engaging the RNS.

The metal insert may be configured to receive a RNS of a pre-filled syringe. In particular, the metal insert preferably may be constructed such that the RNS can be arranged in a free inner volume of the cylindrical metal insert. Flaps protruding axially inward of the metal insert, in particular from an inner surface of said free inner volume, can be used for engaging the RNS.

According to a further aspect of the present invention a cap of a drug delivery device may be provided. The cap may comprise a base and a tubular projection projecting from the base, with the metal insert as described herein inserted therein, with the barbs of the metal insert engaging with an inner wall of the tubular projection. All features and advantages described above with respect to the metal insert can also be provided by a cap comprising said metal insert.

One or more recesses may be provided in the inner wall of the tubular projection for corresponding engagement with the respective barbs of a corresponding one of the one or more projections of the metal insert. The recesses may be provided as through-holes in the inner wall. Preferably, for each of the projections of the metal insert, a respective recess is provided in the inner wall. Also preferred, at least one, preferably all, of said recesses comprise a circumferential extension such that barbs provided at circumferentially opposing ends of the respective projection just can engage into said recess. An especially firm fixation of the metal insert in the tubular projection can thereby be provided.

A RNS may be inserted into the metal insert and with at least some of the flaps of the metal insert engaging an outer wall of the RNS. An especially secure removal of the RNS when removing the cap can thereby be provided, especially by the metal insert arranged in the cap and its mutual connection to the cap by the barbs of the projections, and the RNS by the flaps, respectively.

According to a further aspect of the present invention the drug delivery device may comprise a cap as described herein, with a pre-filled syringe arranged in the drug delivery device and its RNS being received in the metal insert of the cap. All features and advantages described above with respect to the cap can also be provided by a cap comprising said metal insert. As the cap comprises the metal insert according to the present invention, also all features and advantages described above with respect to the metal insert can also be provided by a drug delivery device comprising said metal insert in its respective cap.

According to the present disclosure, a drug or a medicament is a pharmaceutical formulation that contains one or more active pharmaceutical ingredients. An active pharmaceutical ingredient is a substance that is biologically active or has a biological effect on humans or animals. It is used in the diagnosis, mitigation, cure, treatment or prevention of a medical condition or to otherwise affect the structure or function of the body of humans or animals.

Active pharmaceutical ingredients, medicaments and drugs that may be contained and/or dispensed by the mechanisms and devices according to the present disclosure, such as the mechanism and device described herein, are, inter alia, described in handbooks such as Merck Index, for example 15th edition, Rote Liste, such as Rote Liste 2023, the publication Approved Drug Products with Therapeutic Equivalence Evaluations (Orange Book) by the U.S. FDA and the Purple Book Database of Licensed Biological Products, also by the U.S. FDA, all of which are freely accessible via the Internet.

Active pharmaceutical ingredients, medicaments and drugs that may be contained and/or dispensed by the mechanisms and devices according to the present disclosure, such as the mechanism and device described herein, may be selected from: analgesics and antirheumatics (main group 05 of Rote Liste); anti-obesity drugs (main group 06 of Rote Liste); antiallergics (main groups 07 of Rote Liste); antianemics (main group 08 of Rote Liste); antibiotics (main group 10 of Rote Liste); antidiabetic drugs (main group 12 of Rote Liste); antidote medications (main group 13 of Rote Liste); antihemorrhagics (antifibrinolytics and other hemostatic agents) (main group 16 of Rote Liste); antihypoglycemic drugs (main group 18 of Rote Liste); antihypotonics and agents for shock therapy (main group 19 of Rote Liste); anticoagulants (main group 20 of Rote Liste); beta-receptor blockers, calcium channel blockers and inhibitors of the renin-angiotensin-aldosterone system (main group 27 of Rote Liste); bronchospasmolytics/antiasthmatics and other agents for the respiratory tract (main group 28 of Rote Liste); dermatics (main group 32 of Rote Liste); diagnostic agents and agents for diagnostic preparation (main group 35 of Rote Liste); enzyme inhibitors, preparations for enzyme deficiency and transport proteins (main group 40 of Rote Liste); gene and cell therapeutics and RNA interference therapeutics (main group 42 of Rote Liste); hypophysial and hypothalamic hormones, other regulatory peptides, their inhibitors and analogues (main group 50 of Rote Liste); immunomodulators (main group 51 of Rote Liste); lipid-lowering drugs (main group 58 of Rote Liste); migraine medication (main group 61 of Rote Liste); neuropathy preparations and other neurotropic agents (main group 66 of Rote Liste); ophthalmics (main group 67 of Rote Liste); osteoporosis agents, calcium/bone metabolism regulators (main group 68 of Rote Liste); psychotropic drugs (main group 71 of Rote Liste); sera, immunoglobulins and vaccines (main group 75 of Rote Liste); sex hormones and their inhibitors (main group 76 of Rote Liste); vitamins (main group 84 of Rote Liste); oncology drugs, cytostatics, other antineoplastic agents and protectives (main group 86 of Rote Liste); anaesthetics; autoimmune disorder medication, such as rheumatoid arthritis medication and/or multiple sclerosis medication; and/or cardiovascular disease medication.

In a specific example, an active ingredient of a medicament delivered by the mechanisms and devices according to the present disclosure, such as the mechanism and device described herein, is an antirheumatic.

In a further specific example, an active ingredient of a medicament delivered by the mechanisms and devices according to the present disclosure, such as the mechanism and device described herein, is an anti-obesity drug.

In a further specific example, an active ingredient of a medicament delivered by the mechanisms and devices according to the present disclosure, such as the mechanism and device described herein, is an antiallergic.

In a further specific example, an active ingredient of a medicament delivered by the mechanisms and devices according to the present disclosure, such as the mechanism and device described herein, is an antibiotic.

In a further specific example, an active ingredient of a medicament delivered by the mechanisms and devices according to the present disclosure, such as the mechanism and device described herein, is an antidiabetic drug.

In a further specific example, an active ingredient of a medicament delivered by the mechanisms and devices according to the present disclosure, such as the mechanism and device described herein, is an antihypoglycemic drug.

In a further specific example, an active ingredient of a medicament delivered by the mechanisms and devices according to the present disclosure, such as the mechanism and device described herein, is an enzyme inhibitor. In a further specific example, an active ingredient of a medicament delivered by the mechanisms and devices according to the present disclosure, such as the mechanism and device described herein, is a preparation for enzyme deficiency. In a further specific example, an active ingredient of a medicament delivered by the mechanisms and devices according to the present disclosure, such as the mechanism and device described herein, is a transport protein.

In a further specific example, an active ingredient of a medicament delivered by the mechanisms and devices according to the present disclosure, such as the mechanism and device described herein, is a gene therapeutic. In a further specific example, an active ingredient of a medicament delivered by the mechanisms and devices according to the present disclosure, such as the mechanism and device described herein, is a cell therapeutic. In a further specific example, an active ingredient of a medicament delivered by the mechanisms and devices according to the present disclosure, such as the mechanism and device described herein, is an RNA interference therapeutics.

In a further specific example, an active ingredient of a medicament delivered by the mechanisms and devices according to the present disclosure, such as the mechanism and device described herein, is a hypophysial hormone. In a further specific example, an active ingredient of a medicament delivered by the mechanisms and devices according to the present disclosure, such as the mechanism and device described herein, is a hypothalamic hormone. In a further specific example, an active ingredient of a medicament delivered by the mechanisms and devices according to the present disclosure, such as the mechanism and device described herein, is a regulatory peptide other than a hypophysial hormone or a hypothalamic hormone.

In a further specific example, an active ingredient of a medicament delivered by the mechanisms and devices according to the present disclosure, such as the mechanism and device described herein, is an inhibitor of a hypophysial hormone. In a further specific example, an active ingredient of a medicament delivered by the mechanisms and devices according to the present disclosure, such as the mechanism and device described herein, is an inhibitor of a hypothalamic hormone. In a further specific example, an active ingredient of a medicament delivered by the mechanisms and devices according to the present disclosure, such as the mechanism and device described herein, is an inhibitor of a regulatory peptide other than a hypophysial hormone or a hypothalamic hormone.

In a further specific example, an active ingredient of a medicament delivered by the mechanisms and devices according to the present disclosure, such as the mechanism and device described herein, is an analogue of a hypophysial hormone. In a further specific example, an active ingredient of a medicament delivered by the mechanisms and devices according to the present disclosure, such as the mechanism and device described herein, is an analogue of a hypothalamic hormone. In a further specific example, an active ingredient of a medicament delivered by the mechanisms and devices according to the present disclosure, such as the mechanism and device described herein, is an analogue of a regulatory peptide other than a hypophysial hormone or a hypothalamic hormone.

In a further specific example, an active ingredient of a medicament delivered by the mechanisms and devices according to the present disclosure, such as the mechanism and device described herein, is an immunomodulator.

In a further specific example, an active ingredient of a medicament delivered by the mechanisms and devices according to the present disclosure, such as the mechanism and device described herein, is a lipid-lowering drug.

In a further specific example, an active ingredient of a medicament delivered by the mechanisms and devices according to the present disclosure, such as the mechanism and device described herein, is an osteoporosis agent.

In a further specific example, an active ingredient of a medicament delivered by the mechanisms and devices according to the present disclosure, such as the mechanism and device described herein, is a calcium metabolism regulator.

In a further specific example, an active ingredient of a medicament delivered by the mechanisms and devices according to the present disclosure, such as the mechanism and device described herein, is a bone metabolism regulator.

In a further specific example, an active ingredient of a medicament delivered by the mechanisms and devices according to the present disclosure, such as the mechanism and device described herein, is a sex hormone.

In a further specific example, an active ingredient of a medicament delivered by the mechanisms and devices according to the present disclosure, such as the mechanism and device described herein, is an inhibitor of a sex hormone.

In a further specific example, an active ingredient of a medicament delivered by the mechanisms and devices according to the present disclosure, such as the mechanism and device described herein, is an autoimmune disorder medication.

In a further specific example, an active ingredient of a medicament delivered by the mechanisms and devices according to the present disclosure, such as the mechanism and device described herein, is a rheumatoid arthritis medication.

In a further specific example, an active ingredient of a medicament delivered by the mechanisms and devices according to the present disclosure, such as the mechanism and device described herein, is a multiple sclerosis medication.

In a further specific example, an active ingredient of a medicament delivered by the mechanisms and devices according to the present disclosure, such as the mechanism and device described herein, is a cardiovascular disease medication.

The drug or medicament may have, for example, one or more active pharmaceutical ingredients selected from one or more of: carbohydrates and polysaccharides; polypeptides, peptides and proteins (e.g., antibodies, antibody fragments, hormones, growth factors, and enzymes); small molecules with a molecular weight of 500 Da or less; and nucleic acids, double or single stranded DNA (including cDNA and naked DNA), RNA, antisense nucleic acids, such as antisense DNA and RNA, small interfering RNA (siRNA), ribozymes, genes, and oligonucleotides. Nucleic acids may be included into molecular delivery systems such as liposomes, vectors, or plasmids.

The drugs or medicaments contained in or dispensed with the mechanisms according to the present disclosure, such as the mechanism and device described herein, may comprise active pharmaceutical ingredients used in the treatment and/or mitigation and/or prevention and/or cure and/or diagnosis of many different types of medical conditions. These may include, inter alia, one or more of the following conditions: acute coronary syndrome, angina, myocardial infarction, anaphylactic shock, atherosclerosis, diabetes mellitus or complications associated with type 1 or type 2 diabetes mellitus such as diabetic retinopathy, cancer, macular degeneration, inflammation, hay fever, rheumatoid arthritis, thromboembolism disorders such as deep vein or pulmonary thromboembolism, infertility, growth disorder, migraine, autoimmune disorders, multiple sclerosis, osteoporosis, allergies, obesity, and/or cardiovascular diseases.

With a specific example, the condition may be diabetes mellitus.

With another specific example, the condition may be a complication associated with type 1 diabetes mellitus. With another specific example, the condition may be a complication associated with type 2 diabetes mellitus. With another specific example, the condition may be diabetic retinopathy.

With another specific example, the condition may be an inflammation.

With another specific example, the condition may be rheumatoid arthritis.

With another specific example, the condition may be infertility.

With another specific example, the condition may be growth disorder.

With another specific example, the condition may be migraine.

With another specific example, the condition may be an autoimmune disorder.

With another specific example, the condition may be multiple sclerosis.

With another specific example, the condition may be osteoporosis.

With another specific example, the condition may be an allergy.

With another specific example, the condition may be obesity.

With another specific example, the condition may be a cardiovascular disease.

For example, the active pharmaceutical ingredients for the therapy of type 1 or type 2 diabetes mellitus or complications associated with type 1 or type 2 diabetes mellitus include an insulin, such as, human insulin, or a human insulin analogue or derivative, a glucagon-like peptide (GLP-1), GLP-1 analogues or GLP-1 receptor agonists, or an analogue or derivative thereof, a dipeptidyl peptidase-4 (DPP4) inhibitor, or a pharmaceutically acceptable salt or solvate thereof, or any mixture thereof.

The active pharmaceutical ingredient may also be a hormone, such as a hypophysis hormone or a hypothalamus hormone or a regulatory active peptide and an antagonist of a regulatory active peptide. The hormone may be a fertility hormone, a gonadotropine, a growth hormone, a steroid hormone, such as testosterone or oestrogen, a parathyroid hormone, such as teriparatide, epinephrine or a follicle-stimulating hormone.

The active pharmaceutical ingredient may also be an oligonucleotide.

The active pharmaceutical ingredient may also be an antibody. The term "antibody" includes an immunoglobulin molecule or an antigen-binding portion, such as a fragment antigen-binding region, of an immunoglobulin molecule, an antigen-binding molecule based on tetravalent bispecific tandem immunoglobulins, and/or a dual variable region antibody-like binding protein having cross-over binding region orientation (CODV). The antibody may be a monoclonal, a polyclonal, a recombinant, or a chimeric antibody. It may be a de-immunized or humanized, a fully human, or a non-human, such as a murine, antibody. The antibody may be a single chain antibody.

The active pharmaceutical ingredient may also be a polysaccharide, such as a glucosaminoglycane, a hyaluronic acid, a heparin, a low molecular weight heparin or an ultra-low molecular weight heparin or a derivative thereof. The active pharmaceutical ingredient may also be a sulphated polysaccharide, e.g. a poly-sulphated form of the above-mentioned polysaccharides, and/or a pharmaceutically acceptable salt thereof.

The drug or medicament contained in and/or dispensed with the mechanisms and devices according to the present disclosure, such as the mechanism and device described herein, may include one or more of the following active pharmaceutical ingredients: abatacept, adalimumab, aflibercept, alirocumab, anakinra, apomorphine, aripiprazole, belimumab, benralizumab, betamethasone, bimekizumab, brodalumab, brolucizumab, buprenorphine, certolizumab pegol, choriogonadotropin alfa, cyanocobalamin, carbepoetin alfa, denosumab, dulaglutide, dupilumab, enoxaparin sodium, epinephrine, epoetin, epoetin alfa, epoetin beta, epoetin theta, epoetin zeta, erenumab, etanercept, evolocumab, exenatide, filgrastim, follitropin, follitropin alfa, follitropin delta, folic acid, fondaparinux, fremanezumab, fulvestrant, gadobenic acid, gadobutrol, gadodiamide, gadoteric acid, gadoxetic acid, galcanezumab, ganirelix, glatiramer, glucagon, golimumab, goserelin, guselkumab, heparin, icatibant, inclisiran, infliximab, inotersen, insulin aspart, insulin degludec, insulin detemir, insulin glargine, insulin glulisine, insulin human, insulin human-isophane, insulin lispro, insulin isophane, interferon beta-1a, ixekizumab, lanadelumab, lanreotide, lebrikizumab, leuprorelin, liraglutide, lonapegsomatropin, lutropin alfa, menotropin, mepolizumab, methotrexate, methoxy-polyethylene glycol-epoetin beta, mirikizumab, moroctocog alfa, natalizumab, nirsevimab, ofatumumab, omalizumab, paliperidone, pegfilgrastim, peginterferon alfa-2a, peginterferon beta-1a, pegvaliase, propofol, pyridoxine, ranibizumab, rho(D) immune globulin, risankizumab, romosozumab, ropeginterferon alfa-2b, sarilumab, satralizumab, secukinumab, semaglutide, somapacitan, somatrogon, somatropin, sumatriptan, teriparatide, tezepelumab, tildrakizumab, tinzaparin sodium, tocilizumab, tralokinumab, triptorelin, ustekinumab, vedolizumab, volanesorsen, and vutrisiran.

Additionally, or alternatively, the drug or medicament contained in and/or dispensed with the mechanisms and devices according to the present disclosure, such as the mechanism and device described herein, may include one or more of the following active ingredients: Lixisenatide, Taspoglutide, Albiglutide, Efpeglenatide, GLP-1 Eligen, Nodexen, Pegapamodtide, Tirzepatide, and Bamadutide.

Additionally, or alternatively, the drug or medicament contained in and/or dispensed with the mechanisms and devices according to the present disclosure, such as the mechanism and device described herein, may include one or more of the following active ingredients: Mipomersen sodium, Linagliptin, Vildagliptin, Sitagliptin, Denagliptin, Saxagliptin, Berberine, Lutropin, Choriongonadotropin, Desmopressin, Terlipressin, Gonadorelin, Buserelin, Nafarelin, Hylan G-F 20.

Additionally, or alternatively, the drug or medicament contained in and/or dispensed with the mechanisms and devices according to the present disclosure, such as the mechanism and device described herein, may include as an active ingredient a piperidine alkaloid, such as azimine, azithromycine, azcarpine, and/or carpaine.

The drug or medicament may also contain one or more of the following: pharmaceutically acceptable solvents or salts of the active pharmaceutical ingredients described herein; analogues and derivates of the active pharmaceutical ingredients described herein; biosimilars of the active pharmaceutical ingredients described herein; and pharmaceutically acceptable carriers of the active pharmaceutical ingredients described herein.

The drug or medicament contained in and/or dispensed with the mechanisms and devices according to the present disclosure, such as the mechanism and device described herein, may include one or more of the following active pharmaceutical ingredients: paracetamol (acetaminophen), nonsteroidal anti-inflammatory drugs, such as aspirin, ibuprofen and naproxen, cox-2 inhibitors, such as rofecoxib, celecoxib, and etoricoxib, opioids, such as morphine, codeine, oxycodone, hydrocodone, dihydromorphine, pethidine, alcohols, cannabis, nefopam, flupirtine, ziconotide.

The drug or medicament may be contained in a container from which it is dispensed by an action of a user. The container may, for example, be a cartridge, a reservoir or a syringe. The container may extend between a dispensing end and a rear end of the container. The container may be configured to dispense the drug or medicament at the dispensing end, for example through a pierceable septum. At the rear end, the container may be sealed to prevent the exit of the drug or medicament. For example, the container may be sealed by a flexible and/or displaceable plunger at the rear end. In between the dispensing end and the rear end, the container may have a generally tubular hollow housing.

The container may have a single compartment for storing the drug or medicament. Alternatively, the container may also have more than one compartment, such as two compartments. Different compartments thereby may comprise different substances, such as drugs, medicaments, active ingredients, solvents or carriers. Different compartments may also comprise a part of the medicament or drug in solid form, for example as a powder, such as a lyophilized powder, and another part of the medicament in liquid form. The solid part may, for example, comprise the one or more active pharmaceutical ingredient and/or the liquid part may be a solvent.

The container may be configured to allow a mixing of the contents from at least two of the compartments, such as from all of the compartments, prior to and/or during dispensing of the medicament or drug. For example, the container may be configured to provide a fluid connection between the at least two, such as between all of the compartments, for mixing.

For example, the container may comprise two compartments, one comprising a first part of the drug or medicament in solid form, for example the one or more active ingredients, and the other one comprising a second part of the drug or medicament in liquid form, such as a solvent. The container may be configured to provide a fluid connection between the two compartments and to mix the first and second parts prior or during dispensing. This may reconstitute the drug or medicament from its solid form.

The devices and mechanisms according to the present disclosure, such as the mechanism and device described herein, may be disposable, which means that the container holding the medicament is non-replaceable or non-refillable and the devices are disposed of after having dispensed a last dose from the container. Alternatively, the devices and mechanisms according to the present disclosure, such as the mechanism and device described herein, may be reusable, for example, they may allow to replace a container by a new container or to refill the container.

The devices and mechanisms according to the present disclosure, such as the mechanism and device described herein, may be fixed dose devices and mechanisms that only allow the dispensing of a fixed dose of the medicament or drug. Alternatively, the devices and mechanisms according to the present disclosure, such as the mechanism and device described herein, may be variable dose devices and mechanisms that allow for setting of a dose from a multitude of differing settable doses.

The mechanisms and devices according to the present disclosure, such as the mechanism and device described herein, may comprise a dose definition mechanism that allows a user to set the dose to be dispensed prior to dispensing. The dose thereby may be settable as a fixed dose only, for example for fixed dose devices or mechanisms. The dose also may be settable from a multitude of settable doses, such as for variable dose devices or mechanisms.

The doses may be settable as integer multiples of international units of the drug or medicament. They also may be settable as integer multiples of a fraction of said international units, such as integer multiples of half units.

The devices and mechanisms according to the present disclosure, such as the mechanism and device described herein, may be single dose devices that allow dispensing of a dose from the container only once, so that the container is a single dose container. The devices and mechanisms according to the present disclosure, such as the mechanism and device described herein, also may be multiple dose devices that allow for a sequential dispensing of more than one dose from the same container, so that the container is a multi-dose container.

The devices and mechanisms according to the present disclosure, such as the mechanism and device described herein, may be injection devices, such as needle-based injection devices.

Features and functions that may be implemented in devices and mechanisms according to the present disclosure, such as the mechanism and device described herein, and/or requirements that may be met by those mechanisms and devices are also described in standard ISO 11608-1 *(Needle-based injection systems for medical use* - *Requirements and test methods* - *Part 1: Needle-based injection systems),* such as ISO 11608-1 :2022(E), and standard ISO 11608-5 *(Needle-based injection systems for medical use - Requirements and test methods* - *Part 5: Automated functions),* such as ISO 11608-5:2022(E).

The invention will be described in the following with reference to the Figures and the submitted drawings by way of examples. In the drawings there is shown:
- Fig. 1a to e: various views of an autoinjector;
- Fig. 1f: view of a pre-filled syringe for an autoinjector of Fig. 1a to e;
- Fig. 2a to f: various views of a cap of the autoinjector of Fig. 1;
- Fig. 3a to i: various views of a metal insert for a cap of Fig. 2;
- Fig. 4a to g: various views of a needle guard of the autoinjector of Fig. 1;
- Fig. 5a to e: various views of a first type of inner body of the autoinjector of Fig. 1;
- Fig. 6a to d: various views of a spring chassis of the autoinjector of Fig. 1;
- Fig. 7a to d: part sectional views of the inner body and of the spring chassis;
- Fig. 8a to i: various views of a second type of inner body;
- Fig. 9a to j: further views of the spring chassis of Fig. 6, and
- Fig. 10a to c: various views of a component of the housing and the spring chassis with one or more engagement members.

References made in the following regarding directions are made in the context of the drawing and can naturally vary if the viewing position is changed. Moreover, similar parts or parts having similar functions will be referred to in the following using the same feature and/or reference numeral.

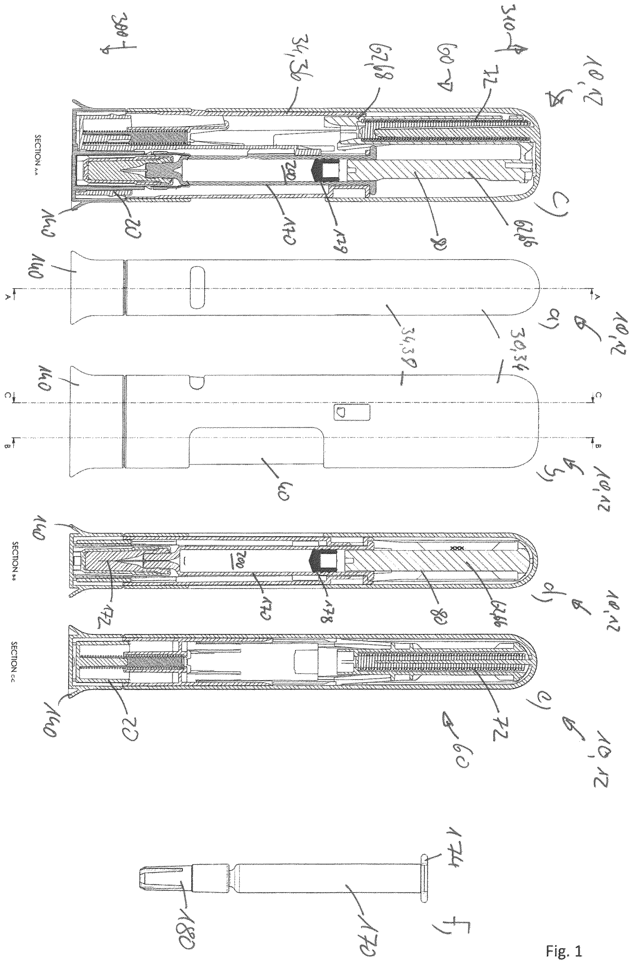

Figs. 1a and 1b show side views of a drug delivery device 10, in particular an autoinjector 12. The autoinjector 12 is a medical instrument that serves the purpose of administering a single dose of medicament 200 (see Fig. 1c). The autoinjector 12 can not only be used by medical staff, but also by a patient to administer the medicament 200 to themselves.

The autoinjector 12 has a housing 30, formed by a body 34, with a syringe window 40 (see Fig. 1b) present therein. In the present embodiment, the housing 30 and body 34, respectively, is provided separated in an outer body 38 (see Fig. 1a, 1b) and an inner body 36 (see Fig. 1c).

A pre-filled syringe 170 is arranged within the housing 30 and visible via the syringe window 40. The pre-filled syringe 170 (see Fig. 1c, f) is filled with the medicament 200.

In the housing 30, a drive mechanism 60 is provided, in the depicted embodiment comprising a general U-shaped spring chassis 62 with a first limb 66 and a second limb 68, see Fig. 1c, 1d, 1e. The first limb 66 acts as piston rod 80, which pushes upon activation a stopper 178 of the pre-filled syringe 170, thereby delivering the medicament 200. The energy needed for said activation is provided by a drive spring 72 which is connected to the second limb 68 of the spring chassis 62.

A needle guard 20 (see e.g. Fig. 1c, e) is arranged at a proximal end 300 of the autoinjector 12. The needle guard 20 has the function of protecting a patient from a needle 172 (see e.g. Fig. 1d) before and after use of the autoinjector 12, i.e. in a storage state and in a lock-out state of the autoinjector 12.

In this connection it should be noted that the terms proximal and distal refer to the position of the needle 172 relative to a patient with proximal meaning closest to a main mass of the body of a patient and distal meaning it is more distant from the main mass of the body of a patient.

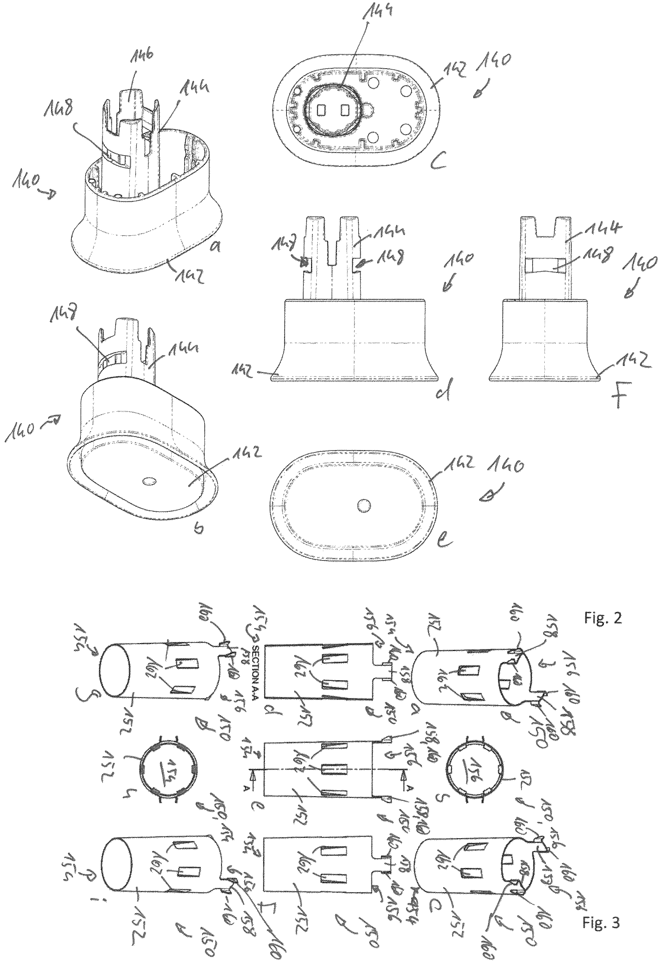

A cap 140 (see also Fig. 2) is arranged at the proximal end 300 of the autoinjector 12 disposed opposite to a distal end 310 of the autoinjector 310. The cap 140 covers both the needle 172 and the needle guard 20 in the storage state of the autoinjector 12.

Figs. 1a and 1b show respective side views from two sides of the autoinjector 12, whereas Figs. 1c to 1e show respective sectional side views of the autoinjector 12, taken along the sectional line A:A of Fig.1a (Fig. 1c), along the sectional line B:B (Fig. 1d), and along the sectional line C:C (Fig. 1e), respectively. Fig. 1f shows a pre-filled syringe 170 with a rigid needle shield (RNS) 180, which can be used in the autoinjector shown in Fig. 1a to 1e.

Figs. 2a to 2f show various views of an example of the cap 140 of the autoinjector 12.

Fig. 2a, b show perspective views of the removable cap 140.

Figs. 2c to 2e show respective side views.

The cap 140 is of single piece design.

As shown in Fig. 2a to 2f, the removable cap 140 has a base 142. The cap 140 tapers outwardly in the region of the base 142 such that the base 142 of the cap 140 has a larger outer diameter than the remaining cap 140. This is particularly beneficial as the base 142 can act as a stand for the autoinjector 12 in the storage state of the autoinjector 12.

A tubular projection 144 projects distally from the base 142 of the cap 140 and is surrounded by an outer wall of the cap 140. The needle guard 20 (see Fig. 1c, e) is configured to cooperate with the cap 140 via one or more snap-fit connections. As depicted, an inner surface of said outer wall of the cap 140 preferably comprises several ribs. These ribs are configured to radially contact the needle guard 20 when this is arranged within the opening 124, wherein the ends of the ribs axially contact the inner body 36 (see Fig. 1c).

The tubular section 144 is constructed to receive the RNS 180 of the pre-filled syringe 170. When the cap 140 is removed for an application of the medicament 200, a simultaneous removal of said RNS 180 has to be ensured.

A possible solution for the problem mentioned in the previous paragraph can be provided by arranging a metal insert 150 as depicted in Fig. 3a to Fig. 3i in the tubular projection 144 of the cap 140. Fig. 3a, 3c, 3g, 3i show perspective views, Fig. 3b, 3h, top and bottom views, respectively, Fig. 3e, 3f side views, and Fig. 3d a sectional view along the line A:A indicated in Fig. 3e.

Said metal insert 150 comprises a cylindrical body 152 having a first end 154 and a second end 156. Further, the metal insert 150 comprising one or more projections 158 projecting axially from the second end 156 of the body 152 and comprising one or more barbs 160 extending radially outwardly.

Preferably, the metal insert 150 is formed from sheet metal.

As depicted, the projections 158 preferably are evenly distributed circumferentially at the second end 156 of the body 152. In the depicted embodiment, two projections 158 are provided which are arranged opposite with respect to each other. Further, the depicted projections 158 comprise two barbs 160 arranged at circumferentially opposing ends of the respective projection 158, wherein additionally the barbs 160 are tapered towards the first end 154.

Referring to Fig. 2a to 2f, if applicable the barbs 160 of the projections 158 are preferably constructed for engaging the recesses 148 provided in the inner surface 146 of the tubular projection of the cap 140. Thereby, a usage of the metal insert 150 with a cap 140 can be provided. An especially firm and secure fixation of the metal insert 150 at the cap 140 can thereby be provided. However, if no recesses 148 are provided, the barbs 160 can also just dig in the material of the cap 140 for a fixation of the metal insert 150.

By arranging the metal insert 150 in the cap 140, both can be used together as part of a drug delivery device 10 such as an autoinjector 12.

Further, an axial height of the metal insert 150 may be greater than an axial width of the insert measured between oppositely disposed barbs 160. This axially elongated shape is similar to the commonly used embodiments of a RNS 180. Especially, with this shape the metal insert 150 is already configured to receive a RNS 180 of a pre-filled syringe 170.

In addition, the metal insert 150 comprises flaps 162 extending radially inwardly towards the first end 154. Said flaps 162 are intended for connecting to the RNS 180, either for a connection to respective features of the RNS 180, or just to dig in the material of the RNS 180.

Different embodiments of the metal insert 150 with respect to the flaps 162 are possible, which can be selected with respect to the expected RNS 180 present at the pre-filled syringe 170.

For instance, the flaps 162 can, as depicted, all be arranged at the same axial height.

Alternatively, two or more groups of flaps 162 are provided, with the different groups of flaps 162 being arranged at different axial heights (not depicted).

Especially, between 3 and 12 metal flaps 162 are provided, in the depicted embodiment 6 flaps 162 are present, see especially Fig. 3b, 3h.

If more than one group of flaps 162 is provided, preferably between 3 and 4 flaps 162 are provided per group of flaps 162.

In all cases, it is preferred that a front end of the flaps 162 is flat with rounded edges.

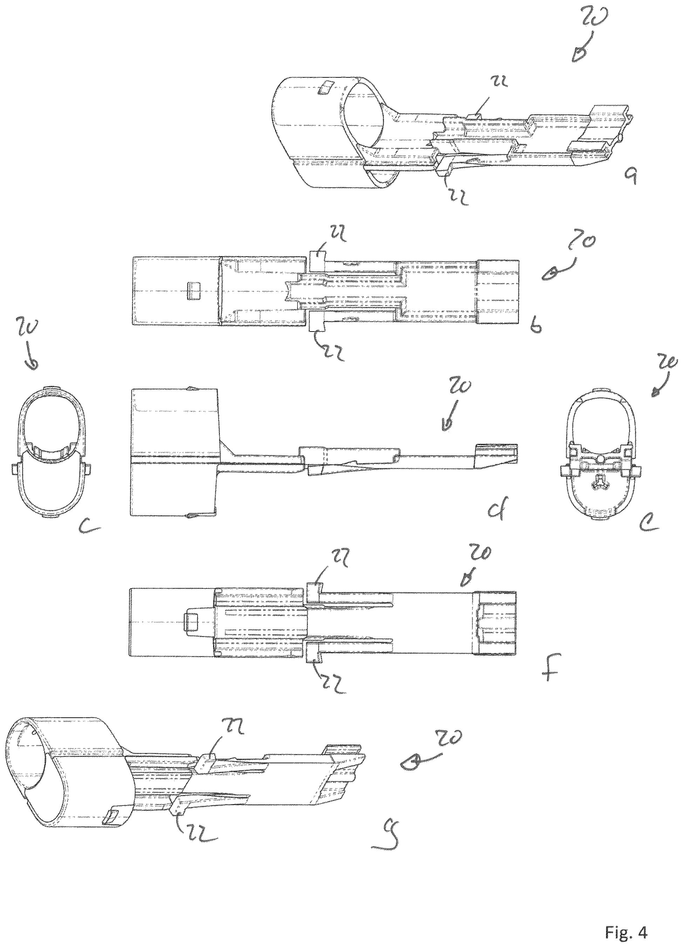

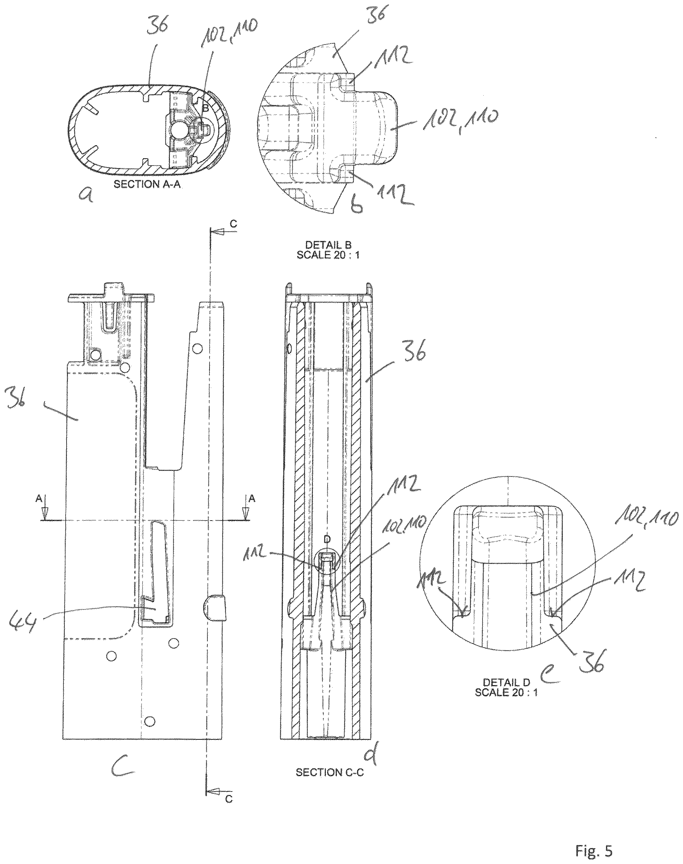

Fig. 4a to 4g show various views of an example of the needle guard 20 of the autoinjector 12, which may be implemented in the autoinjector 12 depicted in Fig. 1. It is configured to cooperate with the inner body 36 of Fig. 5. For this purpose, it is, exemplarily but not limiting, provided with two protrusions 22 cooperating with a respective one of the elongate holes 44 of the inner body 36 (see Fig. 5c).

Figs. 4a and 4g show respective perspective views from above and below of needle guard 20, whereas Figs. 4b, 4d, 4f show respective side views of the needle guard 20., Fig. 4c shows a bottom view, and Fig. 4e shows a top view of the needle guard 20.

Fig. 5a to 5e depict a possible embodiment of an inner body 36, which can form, in particular together with an outer body 38, the body of an autoinjector 12 according to the present invention, see Fig. 1a to 1c.

In particular, said inner body 36 comprises elongated holes 44 for cooperation with the two protrusions 22 of the needle guard 20 depicted in Fig. 4a to 4g.

In addition to taking part in the actual delivery of the medicament 200, the inner body is also part of a mechanism which produces an end of dose click. Said sound provides information about the successful delivery of the medicament 200. In the following, Fig. 5 to 7 are described together with respect to an improvement to said end of dose click mechanism.

Fig. 5 depict an inner body 36, wherein Fig. 5c shows a side view, Fig. 5a a sectional view along the line A:A in Fig. 5c, Fig. 5b an enlarged section of Fig. 5a Fig. 5d a sectional view along the line C:C in Fig. 5c, and Fig. 5e an enlarged section of Fig. 5d,

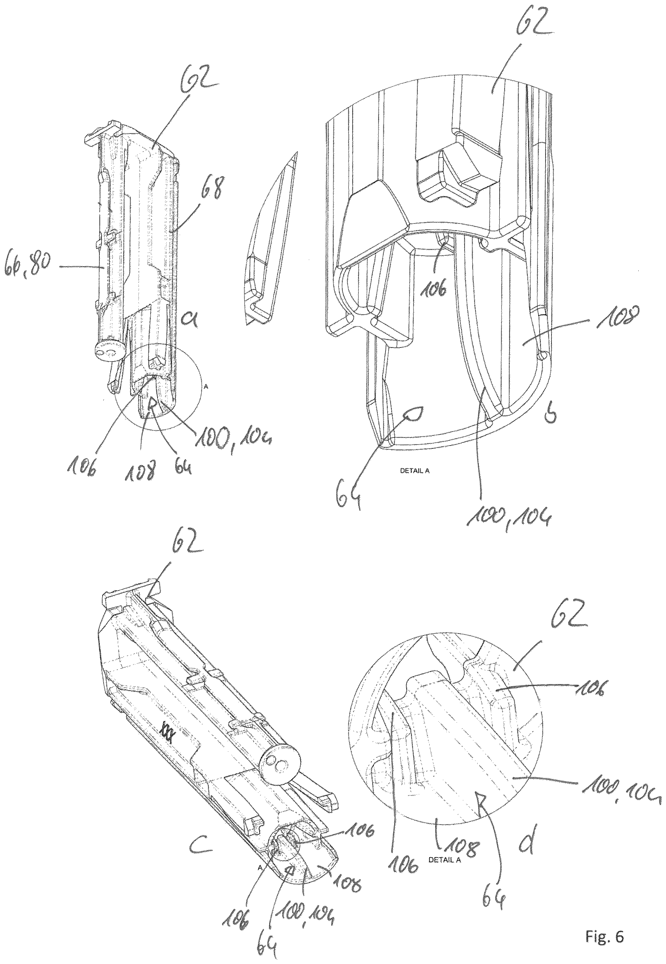

Fig. 6 depict a spring chassis 62, wherein Fig. 6a, 6c show respective perspective views, and Fig. 6b, 6d a respective enlarged section of Fig. 6a, 6c, respectively.

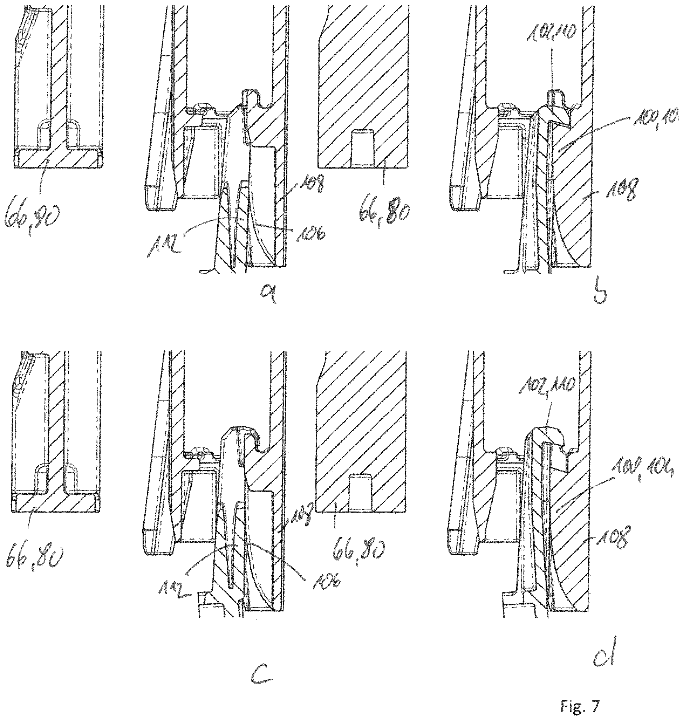

Fig. 7 depict the interaction of the first end of dose click member 100 with the second end of dose click member 102 in sectional views, wherein Fig. 7a, 7c depict a section along further ramp features 106, Fig. 7b, 7d depict a section along an end of dose click ramp 104. Likewise, Fig. 7a, 7b depict a situation immediately after production of the end of dose click sound, Fig. 7c, 7d depict a situation a little later. A detailed description of the process is provided in the following.

In general, a drug delivery device 10, such as for instance an autoinjector (see Fig. 1) comprises an inner body 36 (Fig. 5) and a spring chassis 62 (Fig. 6) moveable relative thereto. In the depicted embodiment, the spring chassis 62 comprises a first end of dose click member 100, namely an end of dose click ramp 104, and the inner body 36 comprises a complementary shaped second end of dose click member 102, namely an end of dose click arm 110, interacting with the first end of dose click member 100. However, in other embodiments this assignment might be vice versa.

Upon dose delivery, the inner body 36 and the spring chassis 62 move with respect to each other, preferably the spring chassis 62 is configured to move towards the inner body 36, and the end of dose click arm 110 travels along the end of dose click ramp 104, which preferably has a curved extent for this purpose. An end of dose click sound is produced when the end of dose click arm 110 reaches the end of the end of dose ramp 104 and snaps behind the end of dose ramp 104, see Fig. 7b.

Likewise to the curved extent of the dose click ramp 104, travelling of the end of dose click arm 110 along the end of dose click ramp can also be enhanced by that the end of dose click arm 110 tapers towards an end thereof, see Fig. 5b, 5d.

However, it is to be noted that the drive spring 72 (not depicted) is often arranged in the vicinity of said end of the end of dose ramp 104, possibly leading to unwanted and in the worst-case harmful interaction of the end of dose arm 110 and the drive spring 72.

Hence, in the present embodiment two further ramp features 106 are provided in addition to the end of dose click ramp 104 that can also interact with the end of dose click arm 110, see Fig. 7a, 7c. Thereby, the end of dose click arm 110 is slightly bend away from the end of the end of dose click ramp 104, thereby leaving a possible arrangement space for the drive spring 72 (not depicted).

In particular, preferably the end of dose click members 100, 102 are configured to make an end of dose click sound before the end of dose click arm 110 contacts one or more further ramp features 106. In other words, the further ramp features 106 do not hinder the process of producing the end of dose click sound.

The aforementioned feature can especially easily be provided in that, as depicted for instance in Fig. 6c, the end of dose click ramp 104 extends further in the axial direction than each of the two further ramp features 106, which are provided one on each side of the end of dose click ramp 104.

Manufacture of the end of dose mechanism can be simplified in that, as depicted in Fig. 6a to 6d, the end of dose click ramp 104 and the two further ramp features 106 are arranged at a common base 108, in particular on an inner surface 64 of the spring chassis 62.

As described above, the end of dose click arm 110 also contacts the further ramp features 106. Hence, the end of dose click arm 110 comprises one or more shoulders 112 for said contact. Preferably, and as depicted for instance in Fig. 5e, for each of the two further ramp features 106, a respective one of two shoulders 112 is provided, wherein each of said shoulders 112 is constructed to run on a respective one of the one or more further ramp features 106.

The above-mentioned enhancement of the end of dose click mechanism can be implemented in a drug delivery device 10 such as an autoinjector 12, for instance in an autoinjector 12 as depicted in Fig. 1. Hence, the respective drug delivery device 10 can comprise a pre-filled syringe 170, and a spring chassis 62 comprising a piston rod 80 acting on a stopper 178 of the pre-filled syringe 170.

Especially said drug delivery device 10 can be configured to emit an end of dose click sound when a medicament 200 stored in the pre-filled syringe 170 is being dispensed from the pre-filled syringe 170.

Even more preferably, the drug delivery device 10 can be configured to emit the end of dose click sound when a medicament 200 stored in the pre-filled syringe 170 is near completely or completely dispensed from the pre-filled syringe 170.

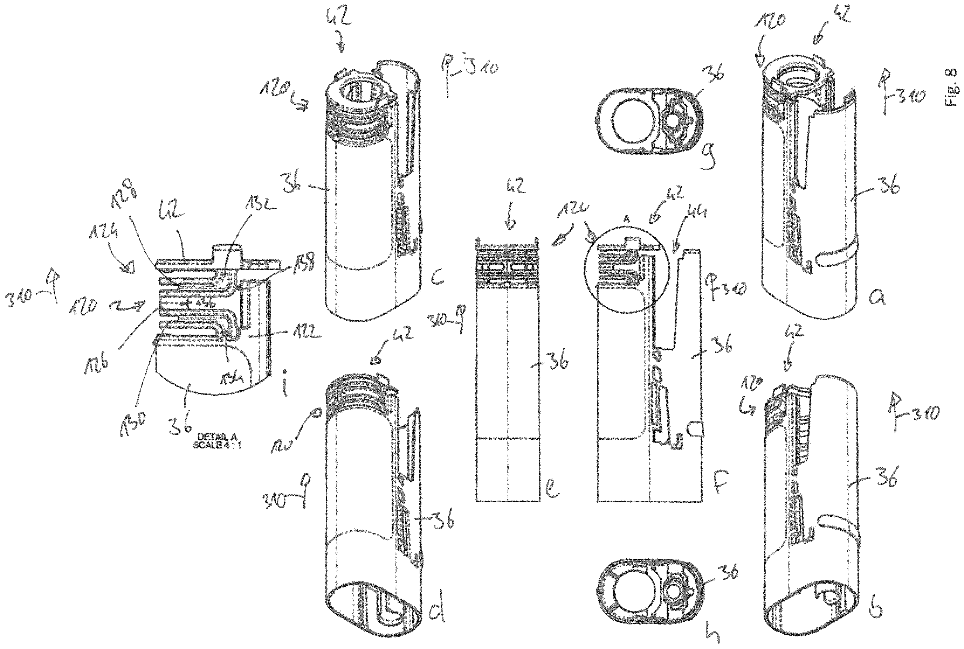

Fig. 8a to 8i depict a second possible embodiment of the inner body 36 of a drug delivery device 10 according to the present invention. However, the depicted embodiment of the inner body 36 is focused on a specific enhancement concerning a force reduction during dose delivery. The above described enhancement concerning the production of the end of dose click sound can also be implemented in the present embodiment, although not explicitly described in the following.

Fig. 8a, 8b, 8c, 8d depict the inner body 36 in different perspective views. Fig. 8e, 8f, show the inner body 36 in different side views, 8g in a top view, and 8h in a bottom view, respectively. Further, Fig. 8i shows an enlarged view of the circular region marked in Fig. 8f.

The depicted inner body 36 can be used in a drug delivery device 10 such as an autoinjector 12, in particular an autoinjector 12 as depicted in Fig. 1. The inner body 36 can be, in combination with an outer body 38 (not depicted), form a body 34 of the autoinjector 12. In the following, all features described with respect to the inner body 36 can also be provided by an accordingly shaped body 34. For the general features and structure of the autoinjector 12 it is referred to the above description of Fig. 1.

In particular, the inner body 36 of the autoinjector is configured to receive a pre-filled syringe 170, wherein a flange 174 of the pre-filled syringe 170 is arranged within the autoinjector 12 at the inner body 36. In the embodiment depicted in Fig. 8, the inner body 36 comprises a force reduction member 120 arranged between the inner body 36 and the pre-filled syringe 170 (not depicted). The force reduction member provides a reduction of forces acting on the syringe 170during delivery of the medicament 200. In summary, a risk of damaging the syringe 170 is thereby reduced.

For the arrangement of the pre-filled syringe 170, the inner body 36 comprises an abutment 42. In other words, the abutment 42 is the dedicated part of the body for an arrangement of the syringe 170, and hence the abutment 42 is configured to receive the pre-filled syringe 170.

Said force reduction member 120 is preferably arranged at the distal end 310 of the inner body 36. The distal end of the inner body 36 is in most of the cases the part of the inner body 36 in contact with the pre-filled syringe 170.

Especially in Fig. 8i, details of a preferred embodiment of the force reduction member 120 are visible.

In particular, force reduction member 120 comprises one or more bridges 138, and additionally a peak force reduction structure 124 and a support 122. This allows to separate different functions of the force reduction member 120 to different parts. For instance, as depicted in Fig. 8i, the peak force reduction structure 124 can be provided as a spring element 126, and the support 122 can be formed by a rigid part of the inner body 36.

Preferably, the one or more bridges 138 is arranged between the peak force reduction structure 124 and the support 122. Thereby, the bridge 138 can act as a pre-determined breaking point, which breaks if a pre-determined force threshold is exceeded.

Preferably, the force reduction member 120 is arranged between the abutment 42 and a main body of the inner body 36..

In the preferred embodiment as depicted in Fig. 8a to 8i, the force reduction member 120 comprises first 128 and second struts 130 connected by a web 136. Further, the first strut 128 is connected to the abutment 42 via two first points of connection 132. In addition, the aforementioned bridge 138 is preferably arranged between the first strut 128 and the support 122.

Likewise, the second strut 130 is connected to the inner body 36 via two second points of connection 134. This can easily be provided, as in the depicted embodiment the web 136 is arranged on a side of the first strut 128 remote from the two first points of connection 132. Further, both struts 128, 130 are U-shaped in shape, allowing easily to provide two points of connection 132, 134 each.

In summary, in the mounted state of the autoinjector 12, the pre-filled syringe 170 is with its flange 174 arranged at the abutment 42. Upon activation, a piston rod 80 acts on a stopper 178 of the pre-filled syringe 170 (see Fig. 1c). A force acting directly onto the syringe 170, in particular on the flange 174 of the syringe 170, is reduced by the force reduction member 120, preferably comprising all features described above.

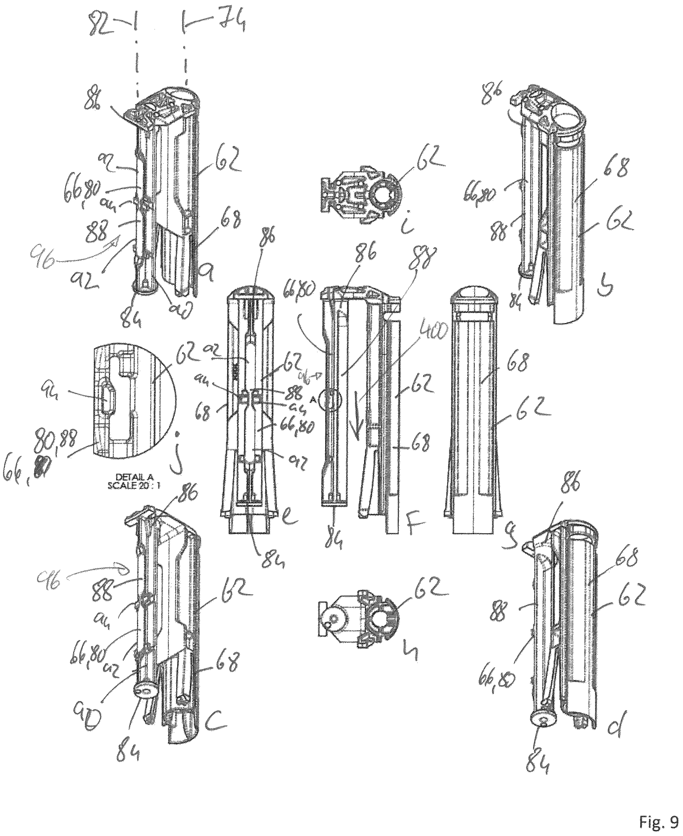

Fig. 9a to 9j depict further views of the spring chassis 62 already shown in Fig. 6a to 6d. Fig 9a to 9d show different perspective views, Fig. 9e to 9g different side views, Fig. 9h a top view, Fig. 9i a bottom view, and Fig- 9j an enlarged view of the circled area marked in Fig. 9f. As the description above of Fig. 6a to 6d was focused on features concerning an enhancement of an end of dose click mechanism, based on Fig. 9a to 9j another preferable feature of the drug delivery device 10, in particular an autoinjector 12, according to the present invention is described.

However, it is nevertheless referred to the features already described above, in particular concerning general features such as for instance the general U-shaped structure of the spring chassis 62 with a first limb 66 forming a piston rod 80 and a second limb 68 for an arrangement of the drive spring 72 (see Fig. 1). In particular, the depicted piston rod 80 comprises a force transmitting end 84, a force receiving end 86 and a connecting portion 88 arranged between the force transmitting end 84 and the force receiving end 86.

As already mentioned, the drug delivery device 10 comprises a reservoir 176 filled with a medicament 200, wherein a stopper 178 is arranged in the reservoir 176. Said reservoir 176 can be provided as part of a pre-filled syringe 170, as shown in Fig. 1f. In particular, the force transmitting end 84 of the piston rod 80 acts on said stopper 178, preferably directly acts on said stopper 178. Further, it is also preferred that the force transmitting end 84 that acts on said stopper 178 has a diameter of more than 80% of said stopper 178.

In a mounted state, the piston rod 80 as part of the spring chassis 62 is stored at least partly within the drug delivery device 10 and guidable in one direction of movement 400 for dispensing a material 200 stored within the drug delivery device 10, see for instance

Fig. 1a to 1e.

During activation, the depicted spring chassis 62 moves within the drug delivery device 10, essentially along one direction of movement 400. As in the depicted embodiment the drug delivery device 10 is spring driven, the drive spring 72 provides the force necessary for the delivery of the drug, wherein the force of the drive spring 72 acts on the piston rod 80, in particular on the force receiving end 86 of the piston rod 80.

Further, as depicted solely in Fig. 9a for a matter of clearness, due to the general U-shape of the spring chassis 62 with its two limbs 66, 68, the piston rod 80 has a piston rod axis 82 and the drive spring 72 at the second limb 68 has a drive spring axis 74, wherein the piston rod axis 82 and the drive spring axis 74 are in parallel to one another and offset from one another with respect to piston rod 80 at the force receiving end 86.

In other words, upon activation, an axial load acts on the piston rod 80 which can lead to buckling of the piston rod 80 and hence may cause bending of the piston rod 80. Due to the offset between drive spring axis 74 and piston rod axis 82, a torque may also act on the piston rod 80, encouraging said buckling and thereby said bending of the piston rod 80. An unwanted and possibly harmful contact of the piston rod 80 with an inner surface of the reservoir 176 may occur.

For avoiding this situation, in spring chassis 62 of the drug delivery device 10 depicted in Fig. 9a to 9j, at least a part of the connecting portion 88 has a reduced cross-sectional area with respect to at least one of the force transmitting end 84 and the force receiving end 86, as depicted even with respect to both ends 84, 86. Hence, even if the piston rod 80 may buckle and hence bend due to an acting axial force and torque during actuation, a contact with the inner surface of the pre-filled syringe 170 can be avoided.

Preferably, the reduced diameter of the connecting portion 88 is reduced by at least about 8% with respect to at least one of the force transmitting end 84 and the force receiving end 86.

In particular, the connecting portion 88 has a radial cut-out 96 defining one or more radial portions with reduced diameter, see in particular Fig. 9a, 9c, and 9f. A radial cut-out 96 in the sense of the present invention is a section of the connecting portion 88, in which a radial extent of the connecting portion 88 is reduced compared to neighboring sections of the connecting portion 88 adjacent to the respective radial cut-out 96.

In addition, the one radial cut-out 96 preferably extends over 30 to 70 % of a length of the piston rod 80, see for instance Fig. 9a. As said buckling and bending mainly occurs at the beginning of the actual delivery, and hence when the piston rod 80 contacts the stopper 178, different positions of the stopper 178 within the syringe 170 corresponding to different filling levels of the syringe 170, can be covered. In addition, if nevertheless a buckling and bending, respectively, occurs during the actual delivery process, avoiding a contact between the piston rod 80 and the syringe 170 can be provided over a wide fraction of the actual movement of the piston rod 80 within the syringe 170.

In particular, as clearly visible for instance in Fig. 9f, the radial cut-out 96 has chamfered ends. As if at all a contact between the piston rod 80 and the syringe 170, or in general the reservoir 176 occur, it mostly will be at one of the ends of the radial cut-out 96. Chamfered ends of the cut-out 96 help prohibiting a sharp rising in acting forces in this case, further protecting the syringe 170.

As nevertheless the piston rod 80 may bend due to the acting torque, the connecting portion 88 may comprises one or more guide elements 90 arranged over its length, wherein further said guide elements 90 may comprise a transverse wall 92 and one or more noses 94 distributed over a length of the connecting portion 88, see especially Fig. 9a, 9c, 9e, and 9j. Another case in which said one of more guide elements 90 can play its strength and advantages is to compensate a bending of the piston rod 80 due to deformation in moulding.

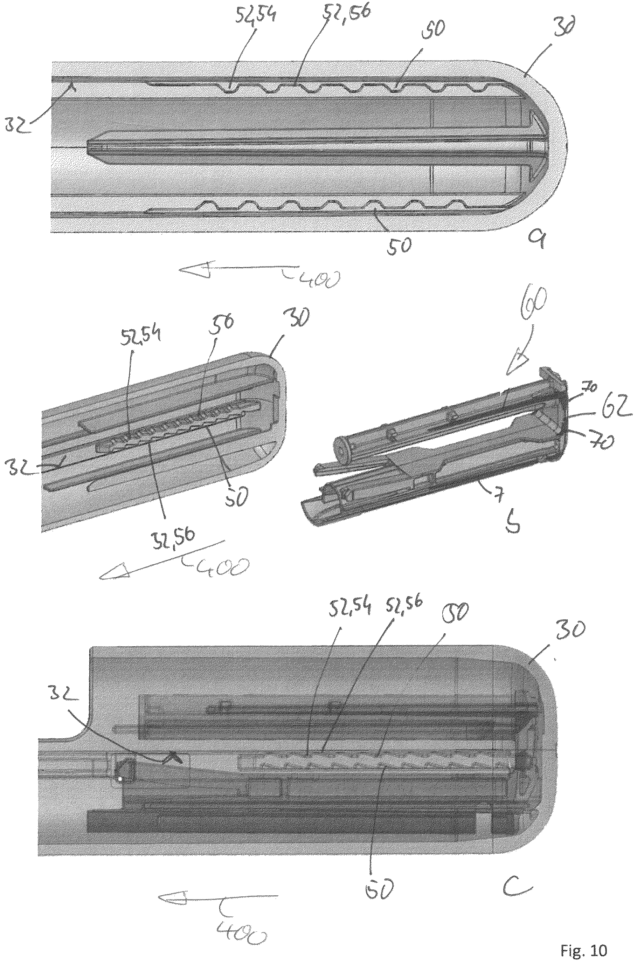

Yet another possible improvement of the drug delivery device 10, such as for example a single use autoinjector 12, according to the present invention is depicted in Fig. 10a to 10c. However, all features and advantages described above with respect to other embodiments are also compatible and may be provided in combination with the respective features depicted in Fig. 10a to 10c.

In particular, Fig. 10a, 10c depict sectional views of a component of the housing 30, whereas Fig. 10b shows on the left a perspective sectional view of the component of the housing 30 of Fig. 10c, and on the right a spring chassis 62 adapted to act together with said component of the housing 30.

As already described in detail above, for instance with respect to Fig. 1a to Fig. 1f, the drug delivery device 10 may comprise a component of the housing 30 in which a drive mechanism 60 (see Fig. 1a to 1e) is stored at least partly within the component of the housing 30 and guidable in one direction of movement 400 for dispensing a material 200 stored within the drug delivery device 10.

In this connection it should be noted that the autoinjector may comprise a speed reduction mechanism. The speed reduction mechanism being configured to generate losses of energy of the spring chassis that slow down the spring chassis as it travels towards proximally during dispense. The spring reduction mechanism being designed to slow down a faster moving spring chassis more than a slower moving spring chassis as the slower moving spring chassis experiences less force losses.

In the embodiment shown embodiment, the component of the housing 30 that acts as the speed reduction mechanism comprises one or more engagement members 50 on an inner surface 32 thereof. In particular, the one or more engagement members 50 respectively facing the drive mechanism 60 and being engagable by an element of the drive mechanism 60. By this, at least some energy, provided for instance by a drive spring 72 (see Fig. 1 c, 1e) is diverted from the movement of the element of the drive mechanism 60. An excessive acceleration of said element of the drive mechanism 60, which might occur especially at the beginning of its movement before even acting for instance on a stopper 178 of a pre-filled syringe 170 arranged in the drug delivery device 10, can thereby be prohibited, in particular preferably by deflecting the element of the drive mechanism 60 transversely and/or radially with respect to said one direction of movement 400 by the one or more engagement members 50.

Preferably, as shown in Fig. 10a to 10c, two engagement members 50 are provided, with the two engagement members 50 being arranged on oppositely disposed inner surfaces 32 of the component of the housing 30. In other words, the two engagement members 50 act on different sides of the element of the drive mechanism 60, thereby providing a more or less chaotic bouncing of the of the element of the drive mechanism 60 within the housing 30. An unwanted acceleration or displacement of the element of the drive mechanism 60 can thereby be avoided.

An especially simple embodiment of said engagement members 50 can be realized in that the engagement members 50 comprise an outer surface 52 having an undulating structure comprising peaks 54 and valleys 56. Preferably, said peaks 54 and valleys 56 are peaks 54 and valleys 56 of a wavelike and/or saw tooth like structure, see Fig. 10a to 10c. A repeated deflection of the element of the drive mechanism 60 can thereby be provided in a mechanical simple way.

Additionally, the peaks 54 and valleys 56 of one of the engagement members 50 are offset with respect to the peaks 54 and valleys 56 of the other one of the engagement members 50 along the one direction of movement 400. In other words, along the one direction of movement 400, a peak 54 on one of the engagement members 50 is met with a valley 56 of the respective other engagement member 50, and vice versa. In particular, this may lead to an oscillation of the element of the drive mechanism 60. An oscillation in the sense of the present disclosure is not necessarily periodic, but might be any movement which comprises alternating acceleration in opposite directions, preferably perpendicular to the one direction of movement 400. However, said oscillation can also be induced without the anti-matched arrangement of peaks 54 and valleys 56. The deflection caused by the respective pair of engagement members 50 is thereby enhanced, which preferably face one another and/or face in opposite directions.

In this connection it should be noted that, as described above, part of the mechanical energy provided to drive the drive mechanism 60 is diverted from the movement of the drive mechanism in the one direction of movement 400. This is of advantage, as the actual dispensing act has not started yet, and said provided mechanical energy, if not diverted, might lead to an excessive large acceleration of the drive mechanism 60. Hence, the one or more engagement members 50 are provided to reduce a speed of the drive mechanism 60 in the one direction of movement 400, in particular before the piston rod 80 contacts the stopper 178 (see Fig. 1c, d).

As depicted on the right side of Fig. 10b, the drive mechanism 60 has one or more complementary shaped counter engagement members 70, such as cut outs, formed at one or more positions corresponding to a position of the one or more engagement members 50. Again, as depicted on the right-hand side in Fig. 10b, especially when the drug delivery device 10 is a spring driven drug delivery device 10 comprising a drive spring 72, the drive mechanism 60 may comprise a spring chassis 62, which in turn carries the counter engagement members 70.

The present disclosure also relates to the following first set of enumerated embodiments:
1. A drug delivery device (10) comprising an inner body (36) and a spring chassis (62) moveable relative thereto, wherein the spring chassis (62) comprises a first end of dose click member (100) and the inner body (36) comprises a complementary shaped second end of dose click member (102) interacting with the first end of dose click member (100), wherein one of the first and second end of dose click members (102) comprises an end of dose click ramp (104) and the other one of the first (100) and second end of dose click members (102) comprises an end of dose click arm (110), wherein one or more further ramp features (106) are provided in addition to the end of dose click ramp (104) that can also interact with the end of dose click arm (110).
2. A drug delivery device (10) comprising an inner body (36) and a spring chassis (62) moveable relative thereto, wherein the spring chassis (62) comprises a first end of dose click member (100) and the inner body (36) comprises a complementary shaped second end of dose click member (102) interacting with the first end of dose click member (100), wherein one of the first and second end of dose click members (102) comprises an end of dose click ramp (104) and the other one of the first and second end of dose click members (102) comprises an end of dose click arm (110), wherein the end of dose click members (100, 102) are configured to make an end of dose click sound before the end of dose click arm (110) contacts one or more further ramp features (106).
3. A drug delivery device (10) according to embodiment 1 or embodiment 2, wherein two further ramp features (106) are provided one on each side of the end of dose click ramp (104).
4. A drug delivery device (10) according to one of embodiments 1 to 4, wherein the end of dose click ramp (104) has a curved extent.
5. A drug delivery device (10) according to one of embodiments 1 to 4, wherein the end of dose click ramp (104) and the one or more further ramp features (106) are arranged at a common base (108).
6. A drug delivery device (10) according to one of embodiments 1 to 5, wherein the end of dose click ramp (104) extends further in the axial direction than each of the one or more further ramp features (106).
7. A drug delivery device (10) according to one of embodiments 1 to 6, wherein the end of dose click ramp (104) and the one or more further ramp features (106) are provided on an inner surface (64) of the spring chassis (62)
8. A drug delivery device (10) according to one of embodiments 1 to 7, wherein the end of dose click arm (110) tapers towards an end thereof.
9. A drug delivery device (10) according to one of embodiments 1 to 8, wherein the end of dose click arm (110) is provided within the inner body (36).
10. A drug delivery device (10) according to one of embodiments 1 to 9, wherein the end of dose click arm (110) comprises one or more shoulders (112).
11. A drug delivery device (10) according to embodiment 10, wherein the one or more shoulders (112) are constructed to run on a respective one of the one or more further ramp features (106).
12. A drug delivery device (10) according to embodiment 10 or 11, wherein the end of dose click arm (110) comprises two shoulders (112).
13. A drug delivery device (10) according to one of embodiments 1 to 12, wherein the spring chassis (62) is configured to move towards the inner body (36).
14. A drug delivery device (10) according to embodiment 13 and one of embodiments 10 to 12, wherein the end of dose click members are configured to make an end of dose click sound before the one or more shoulders (112) of the end of dose click arm (110) contact the one or more further ramp features (106) of the first member.
15. A drug delivery device (10) according to one of embodiments 1 to 14, further comprising a pre-filled syringe (170), with the spring chassis (62) comprising a piston rod (80) acting on a stopper (178) of the pre-filled syringe (170) and the drug delivery device (10) being configured to emit an end of dose click sound when a medicament (200) stored in the pre-filled syringe (170) is being dispensed from the pre-filled syringe (170).
16. A drug delivery device (10) according to embodiment 15, wherein the drug delivery device (10) is configured to emit the end of dose click sound when a medicament (200) stored in the pre-filled syringe (170) is near completely or completely dispensed from the pre-filled syringe (170).

### The present disclosure also relates to the following second set of enumerated embodiments:

1. A drug delivery device (10) comprising a component of the housing (30) in which a drive mechanism (60) is stored at least partly within the component of the housing (30) and guidable in one direction of movement (400) for dispensing a material (200) stored within the drug delivery device (10), wherein the component of the housing (30) comprises one or more engagement members (50) on an inner surface (32) thereof, with the one or more engagement members (50) respectively facing the drive mechanism (60) and being engagable by an element of the drive mechanism (60).
2. A drug delivery device (10) in accordance with embodiment 1, wherein two engagement members (50) are provided, with the two engagement members (50) being arranged on oppositely disposed inner surfaces (32) of the component of the housing (30).
3. A drug delivery device (10) in accordance with embodiment 1 or embodiment 2, wherein the one or more engagement members (50) are configured to deflect the element of the drive mechanism (60) transversely and/or radially with respect to said one direction of movement (400).
4. A drug delivery device (10) in accordance with one of embodiments 1 to 3, wherein the engagement members (50) comprise an outer surface (52) having an undulating structure comprising peaks (54) and valleys (56).
5. A drug delivery device (10) in accordance with embodiment 4, wherein the peaks (54) and valleys (56) are peaks (54) and valleys (56) of a wavelike and/or saw tooth like structure.
6. A drug delivery device (10) in accordance with embodiment 4 or embodiment 5, wherein the peaks (54) and valleys (56) of one of the engagement members (50) are offset with respect to the peaks (54) and valleys (56) of the other one of the engagement members (50) along the one direction of movement (400).
7. A drug delivery device (10) in accordance with one of embodiments 1 to 6, wherein the one or more engagement members (50) are provided to reduce a speed of the drive mechanism (60) in the one direction of movement (400).
8. A drug delivery device (10) in accordance with one of embodiments 1 to 7, wherein the drive mechanism (60) has one or more complementary shaped counter engagement members (70), such as cut outs, formed at one or more positions corresponding to a position of the one or more engagement members (50).
9. A drug delivery device (10) in accordance with one of embodiments 1 to 8, the two oppositely disposed engagement members (50) face one another and/or face in opposite directions.
10. A drug delivery device (10) in accordance with one of embodiments 1 to 9, wherein the drug delivery device (10) is a single use autoinjector.
11. A drug delivery device (10) in accordance with one of the preceding embodiments, further comprising a pre-filled syringe (170), with the pre-filled syringe (170) comprising a stopper (178) and the drive mechanism (60) acting directly on the stopper (178).
12. A drug delivery device (10) in accordance with one of the preceding embodiments, wherein the drug delivery device (10) is a spring driven drug delivery device (10) comprising a drive spring (72).
13. A drug delivery device (10) in accordance with one of embodiments 1 to 14, wherein the drive mechanism (60) comprises a spring chassis (62).
14. A drug delivery device (10) in accordance with embodiments 13, wherein the one or more engagement members (50) are configured to bring about an oscillation of the spring chassis (62) with respect to said one direction of movement (400).
15. A drug delivery device (10) in accordance with embodiment 13 or embodiment 14, wherein the spring chassis (62) comprises a piston rod (80).
16. A drug delivery device (10) in accordance with one of the embodiments 13 to 15, wherein the spring chassis (62) is an at least generally U-shaped spring chassis (62).
17. A drug delivery device (10) in accordance with embodiment 15 and embodiment 16, wherein the piston rod (80) forms one first limb (66) of the U-shaped spring chassis (62).
18. A drug delivery device (10) in accordance with embodiment 12 and one of the embodiments 16 or 17, wherein the drive spring (72) is accommodated in a second limb (68) of the U-shaped spring chassis (62).
19. A drug delivery device (10) in accordance with one of embodiments 16 to 18, wherein a respective engagement member (50) interacts with a respective limb of the at least generally U-shaped spring chassis (62).

### The present disclosure also relates to the following third set of enumerated embodiments:

1. A drug delivery device (10) comprising a piston rod (80) that is stored at least partly within the drug delivery device (10) and guidable in one direction of movement (400) for dispensing a material (200) stored within the drug delivery device (10), the piston rod (80) comprising a force transmitting end (84), a force receiving end (86) and a connecting portion (88) arranged between the force transmitting end (84) and the force receiving end (86), wherein the at least a part of the connecting portion (88) has a reduced cross-sectional area with respect to at least one of the force transmitting end (84) and the force receiving end (86).
2. A drug delivery device (10) according to embodiment 1, wherein the connecting portion (88) has a radial cut-out (96) defining one or more radial portions with reduced diameter.
3. A drug delivery device (10) according to embodiment 1 or embodiment 2, wherein the drug delivery device (10) is spring driven and comprises a drive spring (72) acting on the force receiving end (86).
4. A drug delivery device (10) according to embodiment 3, wherein the piston rod (80) has a piston rod axis (82) and the drive spring (72) has a drive spring axis (74) and the piston rod axis (82) and the drive spring axis (74) are in parallel to one another and offset from one another with respect to piston rod (80) at the force receiving end (86).
5. A drug delivery device (10) according to one of embodiments 1 to 4 further comprising a reservoir (176) filled with a medicament (200) and comprising a stopper (178) arranged in the reservoir (176), wherein the force transmitting end (84) acts on said stopper (178).
6. A drug delivery device (10) according to embodiment 5, wherein the force transmitting end (84) directly acts on said stopper (178).
7. A drug delivery device (10) according to embodiment 5 or embodiment 6 wherein the force transmitting end (84) that acts on said stopper (178) has a diameter of more than 80% of said stopper (178).
8. A drug delivery device (10) according to one of embodiments 5 to 7, further comprising a pre-filled syringe (170) comprising said reservoir (176).
9. A drug delivery device (10) according to one of embodiments 1 to 8, wherein the reduced diameter of the connecting portion (88) is reduced by at least about 8% with respect to at least one of the force transmitting end (84) and the force receiving end (86).
10. A drug delivery device (10) according to one of embodiments 2 to 9, wherein the one radial cut-out (96) extends over 30 to 70 % of a length of the piston rod (80).
11. A drug delivery device (10) according to one of embodiments 2 to 10, wherein the radial cut-out (96) has chamfered ends.
12. A drug delivery device (10) according to one of embodiments 1 to 11, wherein the connecting portion (88) comprises one or more guide elements (90) arranged over its length.
13. A drug delivery device (10) according to embodiment 12, wherein the guide elements (90) comprise a transverse wall (92) and one or more noses (94) distributed over a length of the connecting portion (88).
14. A drug delivery device (10) according to one of the embodiments, wherein the piston rod (80) is an integral part of an at least generally U-shaped spring chassis (62), with the piston rod (80) forming one limb of the U.
15. A drug delivery device (10) according to one of embodiments 3 to 13 and embodiment 14, wherein the drive spring (72) is partly received in the other limb of the U.
16. A drug delivery device (10) according to one of embodiments 1 to 15, comprising the pre-filled syringe (170) filled with material (200).

### The present disclosure also relates to the following fourth set of enumerated embodiments:

1. A metal insert (150) for an autoinjector, the metal insert (150) comprising a cylindrical body (152) having a first end (154) and a second end (156), the metal insert (150) further comprising one or more projections (158) projecting axially from the second end (156) of the body (152) and comprising one or more barbs (160) extending radially outwardly.
2. The metal insert (150) of embodiment 1, wherein the projections (158) are evenly distributed circumferentially at the second end (156) of the body (152).
3. The metal insert (150) of embodiment 1 or embodiment 2, wherein at least one of the one or more projections (158) comprises two barbs (160) arranged at circumferentially opposing ends of the respective projection (158).
4. The metal insert (150) of one of embodiments 1 to 3, wherein the barbs (160) are tapered towards the first end (154).
5. The metal insert (150) of one of embodiments 1 to 4, further comprising flaps (162) extending radially inwardly towards the first end (154).
6. The metal insert (150) of embodiment 5, wherein the flaps (162) are all arranged at the same axial height.
7. The metal insert (150) of embodiment 5, wherein two or more groups of flaps (162) are provided, with the different groups of flaps (162) being arranged at different axial heights.
8. The metal insert (150) of one of embodiments 5 to 7, wherein between 2 and 12 metal flaps (162) are provided.
9. The metal insert (150) of embodiment 7 and embodiment 8, wherein between 2 and 4 flaps (162) are provided per group of flaps (162).
10. The metal insert (150) of one of embodiments 5 to 9, wherein a front end of the flaps (162) is flat with rounded edges.
11. The metal insert (150) of one of embodiments 1 to 10, wherein an axial height of the metal insert (150) is greater than an axial width of the insert measured between oppositely disposed barbs (160).
12. The metal insert (150) of one of embodiments 1 to 11, wherein the metal insert (150) is formed from sheet metal.
13. The metal insert (150) of one of embodiments 1 to 12, wherein the metal insert (150) is configured to be inserted into a cap (140) of an autoinjector.
14. The metal insert (150) of one of embodiments 1 to 13, wherein the metal insert (150) is configured to receive a rigid needle shield (180) (RNS) of a pre-filled syringe (170).
15. A cap (140) of a drug delivery device (10) comprising a base and a tubular projection (144) projecting from the base, with the metal insert (150) of one of embodiments 1 to 14 inserted therein, with the barbs (160) of the metal insert (150) engaging with an inner wall of the tubular projection (144).
16. The cap (140) according to embodiment 15, wherein one or more recesses (148) are provided in the inner wall of the tubular projection (144) for corresponding engagement with the respective barbs (160) of a corresponding one of the one or more projections (158) of the metal insert (150).
17. The cap (140) according to embodiment 15 or embodiments 16, wherein a RNS (180) is inserted into the metal insert (150) and with at least some of the flaps (162) of the metal insert (150) engaging an outer wall (182) of the RNS (180).
18. A drug delivery device (10) comprising a cap (140) according to one of the embodiments 15 to 17, with a pre-filled syringe (170) arranged in the drug delivery device (10) and its RNS (180) being received in the metal insert (150) of the cap (140). List of references

- 10: Drug delivery device
- 12: Autoinjector

- 20: Needle guard ex18
- 22: Protrusion ex166

- 30: Housing ex12
- 32: Inner surface of housing
- 34: Body
- 36: Inner body
- 38: Outer body
- 40: Syringe window ex14
- 42: Abutment
- 44 46: Elongated hole

- 50: Engagement member
- 52: Outer surface of engagement member
- 54: Peak
- 56: Valley

- 60: Drive mechanism exReleaseMechanism40
- 62: Spring chassis exDriveChassis24
- 64: Inner surface of spring chassis
- 66: First limb
- 68: Second limb
- 70: Counter engagement member
- 72: Drive spring
- 74: Drive spring axis

- 80: Piston rod
- 82: Piston rod axis
- 84: Force transmitting end
- 86: Force receiving end
- 88: Connecting portion
- 90: Guide element
- 92: Transverse wall
- 94: Nose
- 96: Cut-out

- 100: First end of dose click member

- 102: Second end of dose click member
- 104: End of dose click ramp
- 106: Further ramp feature
- 108: Common base
- 110: End of dose click arm
- 112: Shoulder

- 120: Force reduction member
- 122: Support
- 124: Peak force reduction structure
- 126: Spring element
- 128: First Strut
- 130: Second Strut
- 132: First point of connection
- 134: Second point of connection
- 136: Web
- 138: Bridge

- 140: Cap ex70
- 142: Base ex100
- 144: Tubular projection exNeedleshieldholder1 04
- 146: Inner surface of tubular projection
- 148: Recess

- 150: Metal insert
- 152: Body of metal insert
- 154: First end of body
- 156: Second end of body
- 158: Projection
- 160: Barb
- 162: Flap

- 170: Pre-filled syringe ex16
- 172: Needle ex34
- 174: Flange
- 176: Reservoir
- 178: Stopper
- 180: Rigid needle shield (RNS)
- 182: Outer wall of RNS
- 200: Material/Medicament
- 300: Proximal end ex 28
- 310: Distal End ex 30
- 400: Direction of movement

## Claims

1. An autoinjector (12) comprising:
a body (34) having a proximal end (300) and a distal end (310), with the body (34) being configured to receive a pre-filled syringe (170) having a needle (172) arranged at a proximal end (300) and a flange (174) arranged at a distal end (310);
wherein the body (34) is configured to receive the pre-filled syringe (170);
wherein the body (34) further comprises a force reduction member (120) arranged between the body (34) and the pre-filled syringe (170).

2. The autoinjector (12) of claim 1, wherein the body (34) comprises an abutment (42).

3. The autoinjector (12) of claim 2, wherein the abutment (42) of the body (34) is configured to receive the pre-filled syringe (170).

4. The autoinjector (12) if one of claims 1 to 3, wherein the body (34) comprises an inner body (36) and an outer body (38).

5. The autoinjector (12) according to claim 1, wherein the force reduction member (120) is arranged at the distal end (310) of the inner body (36).

6. The autoinjector (12) according to one of claims 1 to 5, wherein the force reduction member (120) comprises one or more bridges (138).

7. The autoinjector (12) according to one of claims 1 to 6, wherein the force reduction member (120) further comprises a peak force reduction structure (124) and a support (122).

8. The autoinjector (12) according to claim 6 and claim 7, wherein a respective one of the one or more bridges (138) is arranged between the peak force reduction structure (124) and the support (122).

9. The autoinjector (12) according to one of claims 7 to 8, wherein the peak force reduction structure (124) is a spring element (126).

10. The autoinjector (12) according to one of claims 7 to 9, to one of the claims and 2 or 3, and to claim 4, wherein the force reduction member (120) is arranged between the abutment (42) and a main body of the inner body (36)..

11. The autoinjector (12) according to one of claims 7 to 10, wherein the force reduction member (120) comprises first (128) and second struts (130) connected by a web (136).

12. The autoinjector (12) according to claim 10 and claim 11, wherein the first strut (128) is connected to the abutment (42) via two first points of connection (132).

13. The autoinjector (12) according to claim 11 or claim 12, wherein the web (136) is arranged on a side of the first strut (128) remote from the two first points of connection (132).

14. The autoinjector (12) according to claims 10 and claim 11 or claim 12 or claim 13, wherein the second strut (130) is connected to the body (34) via two second points of connection (134).

15. The autoinjector (12) according to claim 6 and claim 7 and to one of claims 11 to 14, wherein at least one of the one or more bridges (138) is arranged between the first strut (128) and the support (122).

16. The autoinjector (12) according to one of claims 11 to 15, wherein at least one of the first (128) and second struts (130) is U-shaped in geometry.

17. The autoinjector (12) according to one of claims 1 to 16, further comprising the pre-filled syringe (170) arranged within the body (34).
